# FASCICULE DE BREVET EUROPEEN

(11) **EP 4 271 680 B1**
(45) Date de publication et mention de la délivrance du brevet: **16.09.2026**
(21) Numéro de dépôt: 21823962.2
(22) Date de dépôt: 12.11.2021
(51) Int. Cl.: C07F 1/00, A61P 35/00, C07H 23/00

(54) **COMPLEXES D'OR(I) À LIGANDS FLUORÉS DE TYPE CARBÈNES N-HÉTÉROCYCLIQUES ET LEUR UTILISATION COMME AGENTS THÉRAPEUTIQUES**
GOLD (I)-KOMPLEXE MIT FLUORIERTEN N-HETEROCYCLISCHEN CARBENLIGANDEN UND DEREN VERWENDUNG ALS THERAPEUTIKA
GOLD (I) COMPLEXES WITH FLUORINATED N-HETEROCYCLIC CARBENE LIGANDS AND USE THEREOF AS THERAPEUTIC AGENTS

(30) Priorité: 13.11.2020 FR 2011651
(43) Date de publication de la demande: 08.11.2023
(73) Titulaire: Centre National de la Recherche Scientifique, 75016 Paris (FR); Université de Toulouse, 31400 Toulouse (FR); Institut de Recherche pour le Développement (IRD), 13002 Marseille 2 (FR)
(72) Inventeur: GORNITZKA, Heinz, 31077 TOULOUSE Cedex 4 (FR); VALENTIN, Alexis, 31062 TOULOUSE (FR); BOURGEADE-DELMAS, Sandra, 31062 TOULOUSE (FR); HEMMERT, Catherine, 31077 TOULOUSE Cedex 4 (FR)
(74) Mandataire: Santarelli
(86) Numéro de dépôt international: PCT/FR2021/052002
(87) Numéro de publication internationale: WO 2022/101588

(56) Documents cités:
- PRICE GREGORY A ET AL: "Some insights into the gold-catalysed A3-coupling reaction", JOURNAL OF ORGANOMETALLIC CHEMISTRY, ELSEVIER, AMSTERDAM, NL, vol. 846, 21 June 2017 (2017-06-21), pages 251 - 262, XP085154398, ISSN: 0022-328X, DOI: 10.1016/J.JORGANCHEM.2017.06.019
- ZHANG CHEN ET AL: "Cationic and Neutral N -Heterocyclic Carbene Gold(I) Complexes: Cytotoxicity, NCI-60 Screening, Cellular Uptake, Inhibition of Mammalian Thioredoxin Reductase, and Reactive Oxygen Species Formation", vol. 13, no. 12, 23 May 2018 (2018-05-23), DE, pages 1218 - 1229, XP055821746, ISSN: 1860-7179, Retrieved from the Internet <URL:https://api.wiley.com/onlinelibrary/tdm/v1/articles/10.1002%2Fcmdc.201800181> DOI: 10.1002/cmdc.201800181

## Description

### DOMAINE TECHNIQUE

La présente invention concerne le domaine technique général des molécules thérapeutiques.

Plus particulièrement, la présente invention propose de nouveaux complexes d'or(I) à ligands fluorés de type carbènes *N*-hétérocycliques (NHC pour « N-heterocyclic carbenes » en anglais) comme agents anti-oxydants, anti-cancéreux, anti-bactériens et anti-parasitaires. Ces nouveaux complexes sont notamment utiles dans le traitement de la leishmaniose.

### ÉTAT DE LA TECHNIQUE ANTÉRIEURE

Actuellement, l'OMS a classé 20 maladies tropicales négligées (MTN) infectant un milliard de personnes dans les populations à faible revenu dans 149 pays. Parmi ces MTN, la leishmaniose menace plus d'un milliard de personnes principalement dans les régions tropicales comme le Brésil, l'Inde, l'Afrique de l'Est, mais aussi dans les pays riches, notamment autour de la mer Méditerranée. En 2007, la leishmaniose était endémique dans 88 pays et en 2012 dans 98 pays. A noter que le nombre croissant de cas est certainement sous-estimé car la déclaration de cette maladie n'est obligatoire que dans 33/98 pays concernés.

Les leishmanioses sont des maladies infectieuses causées par des parasites du genre Leishmania, qui sont transmises à un mammifère hôte comme, par exemple, l'Homme, le chien, le singe ou le rongeur, lors de la piqûre d'un phlébotome infecté (Phlebotomus sp./Lutzomilla sp.).

La leishmaniose comprend plusieurs formes cliniques parmi lesquelles la leishmaniose viscérale (LV), mortelle si elle n'est pas traitée et causant environ 20 000 à 30 000 décès par an dans le monde. Elle est due au développement de la forme amastigote intracellulaire du parasite, en particulier dans le foie et la rate, et plus précisément dans les cellules mononucléaires de son hôte vertébré. Les deux principales espèces de *Leishmania* responsables de la LV sont *L. infantum* et *L. donovani.*

Concernant le traitement des leishmanioses et notamment de la LV, il existe actuellement peu de médicaments efficaces et sûrs : amphotéricine B, miltéfosine, dérivés antimoniaux, pentamidine et paromomycine. Parmi eux, la miltéfosine est le seul médicament disponible par voie orale. Ces médicaments présentent différents inconvénients. L'amphotéricine B liposomale a un coût prohibitif. Certains présentent des effets secondaires graves comme, par exemple, la néphrotoxicité de l'amphotéricine B, la tératogénicité de la miltéfosine et des arythmies cardiaques et des pancréatites sévères pour les dérivés antimoniaux. Enfin, ces derniers et la miltéfosine montrent un manque d'efficacité croissant dû à l'émergence de parasites résistants. Le fexinidazole, un 5-nitroimidazole, était récemment en phase Il des essais cliniques contre la LV mais a montré clairement un manque d'efficacité et actuellement, il n'y a pas de nouvelle molécule chimique entrée dans les essais cliniques.

La lutte contre la leishmaniose par les approches traditionnelles, telles que le contrôle des vecteurs et le traitement des patients, se heurte principalement à la difficulté d'identifier de nouvelles molécules bon marché capables de soutenir les processus industriels, deuxièmement au développement rapide de la résistance aux médicaments et troisièmement au manque d'accès aux médicaments pour les populations menacées. Ces questions sont au cœur des préoccupations de l'OMS concernant le contrôle des maladies tropicales. Il est nécessaire de renforcer les connaissances fondamentales par des approches originales concernant l'activité d'un groupe prometteur de molécules actives dans le traitement de la leishmaniose.

Ces dernières années, les inventeurs ont travaillé sur le développement de complexes cationiques et neutres de carbène N-hétérocyclique (NHC) d'or(I) pour des applications biomédicales, en particulier pour les maladies parasitaires.

En 2015, Paloque *et al* ont synthétisé et testé des complexes cationiques et neutres d'or(I) à ligand carbène N-hétérocyclique (NHC) fonctionnalisé par une quinoléine **[1].** Le complexe neutre d'or(I) à ligand NHC substitué par un méthyle et une quinoléine (« complexe 6 ») est le plus intéressant des complexes testés avec une concentration inhibitrice médiane (CI50) de 0,96 µM ± 0,55 µM et un index de sélectivité (SI) de 9,84 avec SI = CC50/CI50, CC50 = concentrations de cytotoxicité de 50% sur la lignée cellulaire murine de macrophages J774A.1 et ce, vis-à-vis du stade amastigote de *L*. *infantum.*

Dans Zhang *et al,* 2018 **[2],** neuf complexes neutres d'or(I) à ligand NHC fonctionnalisé par au moins un groupement aryle ont été testés *in vitro* sur les stades promastigote et amastigote axénique de *L. infantum* et comparés à trois médicaments anti-leishmaniens (amphotéricine B, miltéfosine et pentamidine). Le complexe dans lequel le carbène est substitué par un benzyle et un mésityle présente, vis-à-vis du stade amastigote, la valeur de concentration inhibitrice médiane (CI50) la plus faible (0,19 µM) et l'index de sélectivité (SI) le plus élevé (40,29) avec SI = CC50/CI50 où CC50 = concentrations de cytotoxicité de 50% sur la lignée cellulaire murine de macrophages J774A.1

Des complexes cationiques d'or(I) comportant deux ligands NHC substitués par du triclosan ont également été étudiés et testés sur *P. falciparum* et *L. infantum* **[3].** Ces complexes présentent peu ou pas d'activité contre le stade promastigote de *L. infantum.* Par contre, deux de ces complexes référencés « complexe 2a » et « complexe 2e » sont particulièrement efficaces vis-à-vis du stade amastigote axénique de *L. infantum* avec des CI50 respectifs de 0,21 µM et de 0,39 µM.

Des complexes hétérobimétalliques Au(I) et Ru(II) à base de NHC ont également été décrits par Boselli *et al,* 2015 **[4].** Les deux métaux impliqués dans ces complexes sont connus pour leur activité biologique, l'unité contenant le ruthénium(II) présentant également des propriétés de luminescence. Ces complexes ont des activités biologiques bien moindres, comparées à celles des complexes Au(I) à base de NHC correspondants.

Il convient également de souligner que les complexes cationiques et neutres d'or(I) à ligand NHC sont non seulement connus pour leur activité anti-leishmanienne mais également comme agents anti-oxydants, anti-cancéreux, anti-bactériens et anti-parasitaires **[5].**

Enfin, des complexes d'or(I) à ligand NHC substitués par des radicaux fluorés ont déjà été décrits dans l'état de la technique. Par exemple, Perez *et al,* 2019 ont préparé des complexes neutres d'or(I) à ligand NHC substitué par un radical -C₆F₅ (complexe 4c), ces complexes étant décrits comme utiles comme précurseurs catalytiques pour les réactions catalysées par les métaux de frappe **[6].** Des complexes d' or(I) à base de carbène N-hétérocyclique (NHC) utilisés pour le traitement du cancer sont décrit dans [8].

Du fait de leur intérêt thérapeutique et notamment dans le cadre d'un traitement contre la leishmaniose, les inventeurs se sont fixés pour but d'identifier de nouveaux complexes neutres ou cationiques d'or(I) à ligand NHC ne présentant pas les inconvénients des médicaments anti-leishmaniens actuels, faciles de synthèse et présentant une activité et une sélectivité au moins identiques et voire supérieures à celles des médicaments actuels et/ou à celles des complexes neutres ou cationiques d'or(I) à ligand NHC déjà décrits.

### EXPOSÉ DE L'INVENTION

La présente invention permet d'atteindre le but que se sont fixé les inventeurs et de résoudre tout ou partie des inconvénients des médicaments de référence actuellement utilisés dans le traitement de la leishmaniose.

En effet, les travaux des inventeurs ont montré que l'introduction de ligands fluorés dans les complexes d'or(I) à ligands de type NHC permet d'augmenter significativement la sélectivité des complexes ainsi obtenus sur la forme amastigote axénique de *L. infantum* comparée à la sélectivité des complexes neutres ou cationiques d'or(I) à ligand NHC déjà décrits. Il n'était pas du tout évident que l'introduction d'atomes de fluor dans ces complexes ait une telle conséquence.

Les résultats en terme d'activité et de sélectivité des complexes neutres ou cationiques d'or(I) à ligands fluorés de type NHC selon l'invention se situent dans la même gamme que ceux des médicaments de référence actuellement utilisés. Par ailleurs, les complexes, objet de la présente invention, sont aisément obtenus par une synthèse en quelques étapes et une purification facile avec des rendements élevés.

Ainsi, la présente invention concerne un complexe d'or(I) répondant à l'une des formules (I) ou (II) suivantes : 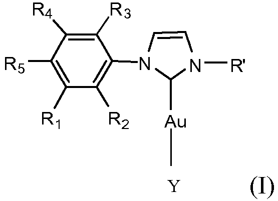 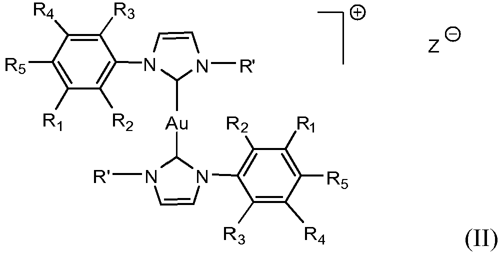 dans lesquelles
- R₁, R₂, R₃, R₄ et R₅, identiques ou différents, sont indépendamment choisis dans le groupe constitué par H, F, CₙF₂ₙ₊₁, OCₙF₂ₙ₊₁ et SCₙF₂ₙ₊₁ avec n égal à 1, 2, 3 ou 4, à condition qu'au moins un parmi R₁, R₂, R₃, R₄ et R₅ soit H et qu'au moins un autre parmi R₁, R₂, R₃, R₄ et R₅ soit choisi dans le groupe constitué par F, CₙF₂ₙ₊₁, OCₙF₂ₙ₊₁ et SCₙF₂ₙ₊₁ ;
- R' représente un groupement alkyle éventuellement substitué ou un groupement aryle éventuellement substitué ;
- Y représente un halogène ou un groupement -S-X avec X représentant un reste d'un ose à 5 ou 6 atomes ou un de ses dérivés ; et
- Z⁻ représente un anion halogénure, un anion nitrate (-NO₃⁻) ou un anion non coordinant,
à condition que, lorsque le complexe d'or(I) répond à la formule (I) et que R₁ = R₂ = R₄ = H, R₃ = R₅ = F et Y = Cl, R' n'est pas un 2,4-difluorophényle.

Il est clair que le complexe d'or(I) de formule (I) dans laquelle R₁ = R₂ = R₄ = H, R₃ = R₅ = F et Y = CI et R' = 2,4-difluorophényle i.e. le chloro-[1,3-bis(2,4-difluorophényle)imidazoline-2-ylidène] or(I) correspondant au complexe 14 décrit dans Price *et al,* 2017 **[7]** est exclu de la portée des complexes d'or(I) selon l'invention. Cette exclusion ne s'applique toutefois pas aux compositions pharmaceutiques contenant de tels complexes ainsi qu'à leur utilisation comme anti-oxydant, comme anti-cancéreux, comme agent anti-bactérien et/ou comme agent anti-parasitaire tel qu'un agent anti-leishmanien.

Lorsque le complexe d'or(I) selon l'invention répond à la formule (I), ce complexe est un complexe neutre d'or(I) à ligand fluoré de type carbène *N-*hétérocyclique. Il peut également être désigné par les expressions « complexe neutre d'or(I) à ligand fluoré de type NHC », « complexe neutre d'or(I) à base de carbène *N-*hétérocyclique fluoré » ou encore « complexe neutre d'or(I) à base de NHC fluoré ». Ces expressions sont équivalentes et utilisables de façon interchangeable.

Lorsque le complexe d'or(I) selon l'invention répond à la formule (II), ce complexe est un complexe cationique d'or(I) comportant deux ligands fluorés de type carbène *N*-hétérocyclique. Il peut également être désigné par les expressions « complexe cationique d'or(I) comportant deux ligands fluorés de type NHC », « complexe cationique d'or(I) à base de bis(carbène *N*-hétérocyclique) fluoré » ou encore », « complexe cationique d'or(I) à base de bis(NHC) fluoré ». Ces expressions sont équivalentes et utilisables de façon interchangeable.

A noter que, typiquement, dans la formule (II), les deux R₁ sont identiques, tout comme les deux R₂, les deux R₃, les deux R₄, les deux R₅ et les deux R'.

Dans les complexes d'or(I) de formule (I) ou (II), R₁, R₂, R₃, R₄ et R₅, identiques ou différents, sont indépendamment choisis dans le groupe constitué par H, F, CF₃, C₂F₅, C₃F₇, C₄F₉, OCF₃, OC₂F₅, OC₃F₇, OC₄F₉, SCF₃, SC₂F₅, SC₃F₇ et SC₄F₉, à condition qu'au moins un parmi R₁, R₂, R₃, R₄ et R₅ soit H et qu'au moins un autre parmi R₁, R₂, R₃, R₄ et R₅ soit choisi dans le groupe constitué par F, CF₃, C₂F₅, C₃F₇, C₄F₉, OCF₃, OC₂F₅, OC₃F₇, OC₄F₉, SCF₃, SC₂F₅, SC₃F₇ et SC₄F₉.

Cette condition i.e. au moins un parmi R₁, R₂, R₃, R₄ et R₅ est un hydrogène exclut de fait le cas où le phényle substituant un des azotes du carbène est un phényle perfluoré, notamment décrit dans **[6].**

Dans un mode de réalisation particulier des complexes d'or(I) de formule (I) ou (II), R₁, R₂, R₃, R₄ et R₅, identiques ou différents, sont indépendamment choisis dans le groupe constitué par H, F, CF₃, OCF₃ et SCF₃, à condition qu'au moins un parmi R₁, R₂, R₃, R₄ et R₅ soit H et qu'au moins un autre parmi R₁, R₂, R₃, R₄ et R₅ soit choisi dans le groupe constitué par F, CF₃, OCF₃ et SCF₃.

Dans un premier mode de réalisation des complexes d'or(I) de formule (I) ou (II), un, deux, trois ou quatre choisis parmi R₁, R₂, R₃, R₄ et R₅ sont un ou des F, le ou les autres étant H.

Dans une première variante de ce premier mode de réalisation, trois choisis parmi R₁, R₂, R₃, R₄ et R₅ sont des F, les autres étant H. Avantageusement, R₂, R₃ et R₅ sont des F.

Dans une seconde variante de ce premier mode de réalisation, deux choisis parmi R₁, R₂, R₃, R₄ et R₅ sont des F, les autres étant H. Avantageusement, R₁ et R₄ sont des F.

Dans une troisième variante de ce premier mode de réalisation, un parmi R₁, R₂, R₃, R₄ et R₅ est un F, les autres étant H. Avantageusement, R₅ est F.

Dans un second mode de réalisation des complexes d'or(I) de formule (I) ou (II), un, deux, trois ou quatre choisis parmi R₁, R₂, R₃, R₄ et R₅ sont un ou des CF₃, le ou les autres étant H.

Dans une première variante de ce second mode de réalisation, trois choisis parmi R₁, R₂, R₃, R₄ et R₅ sont des CF₃, les autres étant H. Avantageusement, R₂, R₃ et R₅ sont des CF₃.

Dans une seconde variante de ce second mode de réalisation, deux choisis parmi R₁, R₂, R₃, R₄ et R₅ sont des CF₃, les autres étant H. Avantageusement, R₁ et R₄ sont des CF₃.

Dans une troisième variante de ce second mode de réalisation, un parmi R₁, R₂, R₃, R₄ et R₅ est un CF₃, les autres étant H. Avantageusement, R₅ est CF₃.

Dans un troisième mode de réalisation des complexes d'or(I) de formule (I) ou (II), un, deux, trois ou quatre choisis parmi R₁, R₂, R₃, R₄ et R₅ sont un ou des OCF₃, le ou les autres étant H.

Dans une première variante de ce troisième mode de réalisation, trois choisis parmi R₁, R₂, R₃, R₄ et R₅ sont des OCF₃, les autres étant H. Avantageusement, R₂, R₃ et R₅ sont des OCF₃.

Dans une seconde variante de ce troisième mode de réalisation, deux choisis parmi R₁, R₂, R₃, R₄ et R₅ sont des OCF₃, les autres étant H. Avantageusement, R₁ et R₄ sont des OCF₃.

Dans une troisième variante de ce troisième mode de réalisation, un parmi R₁, R₂, R₃, R₄ et R₅ est un OCF₃, les autres étant H. Avantageusement, R₅ est OCF₃.

Dans un quatrième mode de réalisation des complexes d'or(I) de formule (I) ou (II), un, deux, trois ou quatre choisis parmi R₁, R₂, R₃, R₄ et R₅ sont un ou des SCF₃, le ou les autres étant H.

Dans une première variante de ce quatrième mode de réalisation, trois choisis parmi R₁, R₂, R₃, R₄ et R₅ sont des SCF₃, les autres étant H. Avantageusement, R₂, R₃ et R₅ sont des SCF₃.

Dans une seconde variante de ce quatrième mode de réalisation, deux choisis parmi R₁, R₂, R₃, R₄ et R₅ sont des SCF₃, les autres étant H. Avantageusement, R₁ et R₄ sont des SCF₃.

Dans une troisième variante de ce quatrième mode de réalisation, un parmi R₁, R₂, R₃, R₄ et R₅ est un SCF₃, les autres étant H. Avantageusement, R₅ est SCF₃.

Par « groupement alkyle », on entend un groupement alkyle, linéaire, ramifié ou cyclique, comprenant de 1 à 15 atomes de carbone, notamment de 1 à 12 atomes de carbone et, en particulier, de 1 à 8 atomes de carbone, ledit groupement alkyle pouvant éventuellement comprendre au moins un hétéroatome et/ou au moins une double ou triple liaison carbone-carbone.

Par « hétéroatome », on entend, dans le cadre de la présente invention, un atome choisi dans le groupe constitué par un azote, un oxygène, un phosphore, un soufre, un silicium, un fluor, un chlore et un brome.

Par « groupement alkyle substitué », on entend, dans le cadre de la présente invention, un groupement alkyle tel que précédemment défini substitué par un groupement ou plusieurs groupements, identiques ou différents, choisi(s) dans le groupe constitué par un halogène; une amine; une diamine; un carboxyle; un carboxylate; un aldéhyde; un ester; un éther; un thioéther; une cétone; un hydroxyle; un alkyle éventuellement substitué; une amide; un sulphonyle; un sulphoxide; un acide sulphonique; un sulphonate; un nitrile; un nitro; un acyle; un époxy; un phosphonate; un isocyanate; un thiol; un glycidoxy et un acryloxy.

Par « halogène », on entend, dans le cadre de la présente invention, un atome choisi dans le groupe constitué par un iode (I), un fluor (F), un chlore (CI) et un brome (Br).

A titre d'exemples particuliers de groupements alkyles éventuellement substitués, utilisables pour R', on peut citer un méthyle, éthyle, n-propyle, isopropyle, butyle, isobutyle, sec-butyle, tert-butyle, pentyle, isopentyle, hexyle, heptyle, octyle et nonyle. Avantageusement, R' est un isopropyle.

Par « groupement aryle », on entend, dans le cadre de la présente invention, tout groupement hydrocarboné comprenant un cycle aromatique ou plusieurs cycles aromatiques, identiques ou différents, liés (i.e fusionnés) ou connectés par une liaison simple ou par une chaîne hydrocarbonée, un cycle aromatique ayant de 3 à 20 atomes de carbone, notamment de 3 à 14 atomes de carbone et, en particulier, de 3 à 8 atomes de carbone et pouvant éventuellement comprendre un hétéroatome.

Par « groupement aryle substitué », on entend, dans le cadre de la présente invention, un groupement aryle tel que précédemment défini substitué par un groupement ou plusieurs groupements, identiques ou différents, choisi(s) dans le groupe constitué par un halogène; une amine; une diamine; un carboxyle; un carboxylate; un aldéhyde; un ester; un éther; un thioéther; une cétone; un hydroxyle; un alkyle éventuellement substitué; une amide; un sulphonyle; un sulphoxide; un acide sulphonique; un sulphonate; un nitrile; un nitro; un acyle; un époxy; un phosphonate; un isocyanate; un thiol; un glycidoxy et un acryloxy.

A titre d'exemples particuliers de groupements aryles éventuellement substitués, utilisables pour R', on peut citer un phényle, biphényle, mésityle, benzyle, naphthyle, quinolyle, pyridine, bipyridine, pyrrole, méthyl-pyrrole, éthyl-pyrrole, thiophénol et le sulfure de méthyle phényle. Avantageusement, R' est un mésityle, un benzyle, un quinolyle, un méthyl-pyrrole, un sulfure de méthyle phényle ou un bipyridine.

Dans les complexes d'or(I) de formule (I) selon l'invention, Y peut être un halogène tel que précédemment défini. Avantageusement, Y est un chlore ou un brome. En particulier, Y est un chlore.

En variante, dans les complexes d'or(I) de formule (I) selon l'invention, Y peut être un groupement -S-X avec X représentant un reste d'un ose à 5 ou 6 atomes de carbone ou un de ses dérivés.

Par « reste d'un ose à 5 ou 6 atomes de carbone », on entend un reste obtenu à partir de l'ose à 5 ou 6 atomes de carbone correspondant par élimination d'un radical hydroxyle. Ainsi, la liaison covalente dans la formule (I) entre S et X implique S et un atome de carbone du reste d'un ose à 5 ou 6 atomes de carbone.

Par « ose à 5 atomes de carbone » également désigné par le terme « pentose », on entend notamment le désoxyribose, le ribose, l'arabinose, le xylose, le lyxose, le ribulose et le xylulose.

Par « ose à 6 atomes de carbone » également désigné par le terme « (desoxy)hexoses », on entend notamment l'allose, l'altrose, le galactose, le glucose, le dextrose, le gulose, l'idose, le mannose, le talose, le fructose, le psicose, le sorbose, le tagatose, le fucose et le rhamnose.

Ces oses peuvent être de configuration L ou D.

Par « dérivé d'un reste d'un ose à 5 atomes de carbone » et « dérivé d'un reste d'un ose à 6 atomes de carbone », on entend un reste d'un ose à 5 atomes de carbone ou à 6 atomes de carbone tel que précédemment défini dans lequel au moins un atome d'hydrogène et/ou au moins un radical hydroxyle d'un reste d'un ose tel que précédemment défini est substitué par un atome ou un groupement chimique tel qu'un atome d'halogène, un groupement alkyle, un groupement hydroxyalkyle, un groupement thioalkyle, groupement sulfhydryle, un groupement acétyle, un groupement silyle, un groupement acyle, un groupement sulfonyle, un groupement amine, un groupement sulfoalkyléther, un groupement sulfate, un groupement phosphate, un groupement carboxyle, un groupement carboxylester, un groupement ammonium quaternaire, un groupement glucosyle, un groupement maltosyle, un groupement chlorotriazinyle ou un groupement ammonium quaternaire.

A titre d'exemple particulier de groupement -S-X mis en œuvre dans les complexes d'or(I) de formule (I), on peut citer le 2,3,4,6-Tetra-O-acétyl-1-thio-β-D-glucopyranosato.

Dans les complexes d'or(I) de formule (II), Z⁻ peut représenter un anion halogénure comme un anion chlorure, un anion fluorure, un anion bromure ou un anion iodure. Avantageusement, Z⁻ représente un anion chlorure ou un anion bromure. En particulier, Z⁻ représente un anion chlorure.

Dans les complexes d'or(I) de formule (II), Z⁻ peut être un anion non coordinant.

Tel qu'utilisée ici, l'expression « anion non coordinant » est utilisée de manière interchangeable avec l'expression « anion faiblement coordinant » et signifie un anion qui soit ne se coordonne pas à un cation ou qui n'est que faiblement coordonné à un cation restant ainsi suffisamment labile pour être déplacé par une base de Lewis neutre. Tout anion non coordinant connu de l'homme du métier est utilisable dans le cadre de la présente invention. Avantageusement, cet anion non coordinant peut être choisi dans le groupe constitué par le tetrafluoroborate (BF₄⁻), hexafluorophosphate (PF₆⁻), benzenesulfonate (CH₅SO₃⁻), bis(trifluorométhanesulfonyl)imide ((N[SO₂(CF₃)]₂)⁻), méthanesulfonate (mesylate, CH₃SO₃⁻), perchlorate (ClO₄⁻), tetrachloroaluminate (AlCl₄⁻), tetrakis[3,5-bis(trifluorométhyl)phényl]borate (B[3,5-(CF₃)₂C₆H₃]₄⁻), tetrakis(hexa-fluoroisopropyl)aluminate (Al[OC(CF₃)₃]₄⁻), tetrakis(pentafluorophényl)borate (B[C₆F₅]₄⁻), p-toluene-sulfonate (tosylate, CH₃C₆H₄SO₂⁻) et le trifluorométhanesulfonate (triflate, CF₃SO₃⁻).

Par ailleurs, le complexe d'or(I) de formule (I) peut être l'un quelconque des complexes listés dans le Tableau 1 ci-après.

La présente invention concerne un complexe d'or(I) selon la présente invention pour utilisation comme médicament. Un tel médicament est utilisable non seulement en médecine humaine mais aussi en médecine vétérinaire. Par conséquent, les sujets susceptibles de recevoir un tel médicament notamment dans le cadre du traitement d'une ou plusieurs pathologies telles que définies ci-après comprennent des animaux, notamment des mammifères et, en particulier, des êtres humains. Des exemples plus particuliers de tels sujets comprennent des êtres humains, des primates non humains, des chiens, des chats, des chevaux, des vaches, des chèvres, des porcs, des moutons, des lapins, des cobayes ou encore des rongeurs.

Ainsi, la présente invention concerne une composition pharmaceutique comprenant, en tant que principe actif, un complexe d'or(I) selon l'invention et/ou le chloro-[1,3-bis(2,4-difluorophényle)imidazoline-2-ylidène] or(I) et un véhicule pharmaceutiquement acceptable.

Par « véhicule pharmaceutiquement acceptable », on entend selon la présente invention, toute substance qui est ajoutée à un complexe d'or(I) selon la présente invention pour favoriser son transport, éviter sa dégradation substantielle dans ladite composition et/ou augmenter sa demi-vie. Avantageusement, un tel véhicule pharmaceutiquement acceptable est stérile et apyrogène. Il est choisi en fonction du type d'application de la composition pharmaceutique de l'invention et notamment en fonction de son mode d'administration.

La composition pharmaceutique selon l'invention est donc constituée par au moins un complexe d'or(I) selon la présente invention et/ou le chloro-[1,3-bis(2,4-difluorophényle)imidazoline-2-ylidène] or(I), sous forme libre ou sous forme d'un sel d'addition avec un acide pharmaceutiquement acceptable, à l'état pur ou sous forme d'une composition dans laquelle il est associé à tout autre produit pharmaceutiquement compatible. Les compositions pharmaceutiques selon l'invention peuvent être employées par voie systémique ; par voie parentérale, par exemple intraveineuse, intra-artérielle, intrapéritonéale, intrathécale, intraventriculaire, intrasternale, intracrânienne, intramusculaire ou sous-cutanée ; par voie topique ; par voie orale ; par voie rectale ; par voie intranasale ou par inhalation.

A titre de compositions solides pour administration orale, on peut utiliser des comprimés, des pilules, des poudres, etc. dans lesquels au moins un complexe d'or(I) selon l'invention et/ou le chloro-[1,3-bis(2,4-difluorophényle)imidazoline-2-ylidène] or(I) est/sont mélangé(s) à un ou plusieurs diluants inertes classiquement utilisés, et éventuellement à d'autres substances telles que, par exemple, un lubrifiant, un colorant, un enrobage etc.

A titre de compositions liquides pour administration orale ou oculaire, on peut utiliser des suspensions, des solutions, des émulsions, des sirops pharmaceutiquement acceptables contenant des diluants inertes classiquement utilisés, et éventuellement d'autres substances comme des produits mouillants, édulcorants, épaississants, etc.

Les compositions stériles pour administration parentérale peuvent être des solutions aqueuses ou non, des suspensions ou des émulsions. Comme solvant ou véhicule, on peut employer l'eau, le propylène-glycol, des huiles végétales ou d'autres solvants organiques convenables. Ces compositions peuvent également contenir des adjuvants, comme des agents mouillants, isotonisants, émulsifiants, etc.

Les compositions pour administration topique peuvent être par exemple des crèmes, lotions, collutoires, gouttes nasales ou oculaires ou aérosol.

L'homme du métier reconnaîtra que la quantité d'un complexe d'or(I) selon l'invention et/ou de chloro-[1,3-bis(2,4-difluorophényle)imidazoline-2-ylidène] or(I), à administrer sera une quantité qui est suffisante pour induire une amélioration des symptômes indésirables à traiter. Une telle quantité peut varier entre autres en fonction de facteurs tels que l'âge, le poids, la condition physique générale du sujet, etc. et peut être déterminée au cas par cas. La quantité peut également varier selon les autres composants d'un protocole de traitement. L'homme du métier reconnaîtra que ces paramètres sont normalement élaborés au cours des essais cliniques. Par ailleurs, l'administration d'un complexe d'or(I) selon la présente invention et/ou du chloro-[1,3-bis(2,4-difluorophényle)imidazoline-2-ylidène] or(I) peut se faire en doses uniques ou fractionnées.

Comme déjà évoqué dans **[5],** les complexes cationiques et neutres d'or(I) à ligand NHC sont non seulement connus pour leur activité anti-leishmanienne mais également comme agents anti-oxydants, anti-cancéreux, anti-bactériens et anti-parasitaires. Les complexes selon l'invention et/ou le chloro-[1,3-bis(2,4-difluorophényle)imidazoline-2-ylidène] or(I) sont donc utiles dans toutes ces applications thérapeutiques.

Dans une première forme de mise en œuvre, le complexe d'or(I) selon l'invention et/ou le chloro-[1,3-bis(2,4-difluorophényle)imidazoline-2-ylidène] or(I) est utile(s) comme agent anti-oxydant. Ainsi, la présente invention concerne un complexe d'or(I) selon la présente invention et/ou le chloro-[1,3-bis(2,4-difluorophényle)imidazoline-2-ylidène] or(I) ou une composition pharmaceutique selon la présente invention pour utilisation dans le traitement et/ou la prévention d'un trouble ou d'une pathologie dans lequel/laquelle sont impliqués des radicaux libres. A titre d'exemples de tels troubles ou de telles pathologies, on peut citer le vieillissement, une maladie coronarienne, le diabète, l'arthrite, l'épilepsie, les accidents vasculaires cérébraux (AVC), la maladie de Parkinson, la maladie d'Alzheimer, une maladie auto-immune et une maladie neurodégénérative.

Dans une deuxième forme de mise en œuvre, le complexe d'or(I) selon l'invention et/ou le chloro-[1,3-bis(2,4-difluorophényle)imidazoline-2-ylidène] or(I) est/sont utile(s) comme agent anti-cancéreux. A noter que cette activité anti-cancéreuse peut être liée, directement ou indirectement, à l'activité anti-oxydante précitée. La présente invention concerne donc un complexe d'or(I) selon la présente invention et/ou le chloro-[1,3-bis(2,4-difluorophényle)imidazoline-2-ylidène] or(I) ou une composition pharmaceutique selon la présente invention pour utilisation dans le traitement et/ou la prévention d'un cancer. A titre illustratif et non limitatif de cancers, on peut citer un mélanome, un cancer colorectal, un cancer du côlon, un Sarcome de Kaposi, un glioblastome, un cancer des ovaires, un cancer du sein, un cancer du foie, un cancer du pancréas, un cancer du poumon, un cancer de la vessie, un cancer de la prostate ou encore des tumeurs neuroendocrines.

Dans une troisième forme de mise en œuvre, le complexe d'or(I) selon l'invention et/ou le chloro-[1,3-bis(2,4-difluorophényle)imidazoline-2-ylidène] or(I) est/sont utile(s) comme agent anti-bactérien. Ainsi, la présente invention concerne un complexe d'or(I) selon la présente invention et/ou le chloro-[1,3-bis(2,4-difluorophényle)imidazoline-2-ylidène] or(I) ou une composition pharmaceutique selon la présente invention pour utilisation dans le traitement et/ou la prévention d'un trouble ou d'une pathologie induit(e) par des bactéries. A titre illustratif et non limitatif de telles bactéries, on peut citer des bactéries Gram- comme *Helicobacter pylori, Acinetobacter baumannii, Enterobacter cloacae, Escherichia coli, Klebsiella pneumoniae* et *Pseudomonas aeruginosa* et des bactéries Gram+ comme *Enterococcus faecium, Enterococcus faecalis, Staphylococcus epidermidis* et *Staphylococcus aureus.*

Dans une quatrième forme de mise en œuvre, le complexe d'or(I) selon l'invention et/ou le chloro-[1,3-bis(2,4-difluorophényle)imidazoline-2-ylidène] or(I) est/sont utile(s) comme agent anti-parasitaire. Ainsi, la présente invention concerne un complexe d'or(I) selon la présente invention et/ou le chloro-[1,3-bis(2,4-difluorophényle)imidazoline-2-ylidène] or(I) ou une composition pharmaceutique selon la présente invention pour utilisation dans le traitement et/ou la prévention d'un trouble ou d'une pathologie induit(e) par des parasites. A titre illustratif et non limitatif de tels parasites, on peut citer les parasites du genre *Trypanosoma* comme *T. brucei, T. gambiense, T. cruzi, T. equiperdum* et *T. congolenze,* et les parasites du genre *Leishmania.*

En effet, dans un mode particulier de réalisation de cette quatrième forme de mise en œuvre, le complexe d'or(I) selon l'invention et/ou le chloro-[1,3-bis(2,4-difluorophényle)imidazoline-2-ylidène] or(I) est/sont utile(s) comme agent anti-leishmanien. Ainsi, la présente invention concerne un complexe d'or(I) selon la présente invention et/ou le chloro-[1,3-bis(2,4-difluorophényle)imidazoline-2-ylidène] or(I) ou une composition pharmaceutique selon la présente invention pour utilisation dans le traitement et/ou la prévention d'une leishmaniose. A titre d'exemples illustratifs et non limitatifs de parasites susceptibles d'induire une telle leishmaniose, on peut citer *L*. *donovani, L. infantum, L. chagasi, L. mexicana, L. amazonensis, L. venezuelensis, L. Tropica; L. major; L.* aethiopica, *L. (Viannia.) braziliensis, L. (V.) guyanensis, L. (V.) panamensis* et *L. (V.) peruvian.*

En d'autres termes encore, la présente invention concerne un procédé pour traiter et/ou prévenir un trouble ou une pathologie tel(le) que précédemment défini(e). Ce procédé consiste à administrer audit sujet une quantité pharmaceutiquement efficace d'un complexe d'or(I) selon la présente invention et/ou de chloro-[1,3-bis(2,4-difluorophényle)imidazoline-2-ylidène] or(I) ou d'une composition pharmaceutique selon la présente invention.

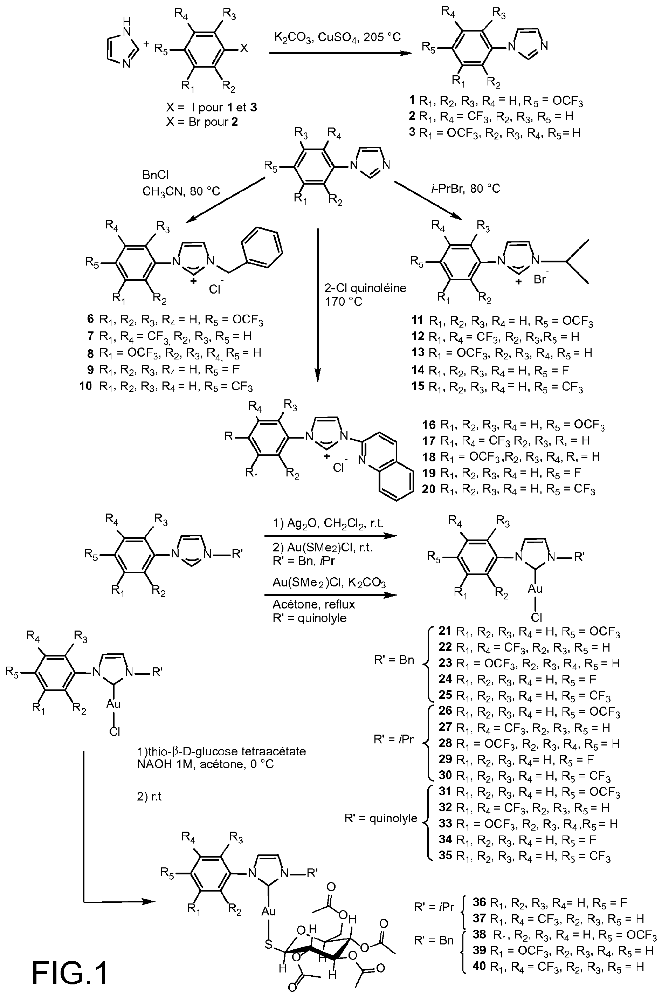

D'autres caractéristiques et avantages de la présente invention apparaîtront encore à l'homme du métier à la lecture des exemples ci-dessous donnés à titre illustratif et non limitatif, en référence à la figure 1 annexée.

### BRÈVE DESCRIPTION DES DESSINS

La Figure 1 est une schématisation de la synthèse des proligands **6-20** et des complexes d'or(I) selon l'invention **21-40.**

### EXPOSÉ DÉTAILLÉ DE MODES DE RÉALISATION PARTICULIERS

### I. Synthèse des complexes selon l'invention.

### I.1. Complexes synthétisés.

Le Tableau 1 ci-dessous liste les différents complexes synthétisés selon l'invention. Ces complexes neutres répondent à la formule (I) telle que précédemment définie avec le groupement Y représentant soit un atome de chlore (« 1 » dans la colonne Y du Tableau 1) soit un groupement 2,3,4,6-Tetra-O-acétyl-1-thio-β-D-glucopyranosato (« 2 » dans la colonne Y du Tableau 1). Le groupement -C₉H₆N correspond à un groupement quinolyle.

**Tableau 1**

| Complexe | -R₁ | -R₂ | -R₃ | -R₄ | -R₅ | -R' | -Y |
|---|---|---|---|---|---|---|---|
| **21** | -H | -H | -H | -H | -OCF₃ | -CH₂-C₆H₅ | 1 |
| **22** | -CF₃ | -H | -H | -CF₃ | -H | -CH₂-C₆H₅ | 1 |
| **23** | -OCF₃ | -H | -H | -H | -H | -CH₂-C₆H₅ | 1 |
| **24** | -H | -H | -H | -H | -F | -CH₂-C₆H₅ | 1 |
| **25** | -H | -H | -H | -H | -CF₃ | -CH₂-C₆H₅ | 1 |
| **26** | -H | -H | -H | -H | -OCF₃ | -CH(CH₃)₂ | 1 |
| **27** | -CF₃ | -H | -H | -CF₃ | -H | -CH(CH₃)₂ | 1 |
| **28** | -OCF₃ | -H | -H | -H | -H | -CH(CH₃)₂ | 1 |
| **29** | -H | -H | -H | -H | -F | -CH(CH₃)₂ | 1 |
| **30** | -H | -H | -H | -H | -CF₃ | -CH(CH₃)₂ | 1 |
| **31** | -H | -H | -H | -H | -OCF₃ | -C₉H₆N | 1 |
| **32** | -CF₃ | -H | -H | -CF₃ | -H | -C₉H₆N | 1 |
| **33** | -OCF₃ | -H | -H | -H | -H | -C₉H₆N | 1 |
| **34** | -H | -H | -H | -H | -F | -C₉H₆N | 1 |
| **35** | -H | -H | -H | -H | -CF₃ | -C₉H₆N | 1 |
| **36** | -H | -H | -H | -H | -OCF₃ | -CH(CH₃)₂ | 2 |
| **37** | -CF₃ | -H | -H | -CF₃ | -H | -CH(CH₃)₂ | 2 |
| **38** | -OCF₃ | -H | -H | -H | -H | -CH₂-C₆H₅ | 2 |
| **39** | -CF₃ | -H | -H | -CF₃ | -H | -CH₂-C₆H₅ | 2 |
| **40** | -H | -H | -H | -H | -F | -CH₂-C₆H₅ | 2 |
| **41** | -H | -H | -H | -H | -SCF₃ | -CH₂-C₆H₅ | 1 |
| **42** | -H | -H | -H | -H | -SCF₃ | -CH(CH₃)₂ | 1 |

### I.2. Principe de la synthèse.

Les imidazoles substitués **1, 2** et **3** ont été préparés par une N-arylation catalysée par le cuivre de l'imidazole avec du 4-(trifluorométhoxy)iodobenzène pour **1,** du 1,3-bis(trifluorométhyl)-5-bromobenzène pour **2** ou du 3-(trifluorométhoxy)iodobenzène pour **3** en présence de K₂CO₃ et CuSO₄.

Les proligands **6-8, 11-13** et **16-18** ont été facilement obtenus après une étape de quaternisation du 1-benzylimidazole pour **6-8,** du 2-bromopropane pour **11-13** et du 2-chloroquinoléine pour **16-18** avec le 1-(4-fluorophényl)-1H-imidazole (Figure 1). Les proligands **9, 10, 14, 15, 19** et **20** ont été aisément obtenus en une étape après une étape de quaternisation du 1-benzylimidazole pour le **9** et **10,** du 2-bromopropane pour le **14** et **15** et de la 2-chloroquinoléine pour le **19** et **20** avec le 1-(4-fluorophényl)-1*H-*imidazole (Figure 1).

Les complexes d'or(I) **21-30** ont été synthétisés via la voie de transmétallation impliquant la base douce Ag₂O, suivie par l'addition ultérieure de Au(SMe₂)Cl (Figure 1). Les complexes d'or(I) **31-35** ont été préparés par métallation directe impliquant K₂CO₃ et Au(SMe₂)Cl (Figure 1). Les complexes d'or(I) **36-40** ont été obtenus par échange de chlorure des complexes **21-23, 27 et 29** avec du tétraacétate de thio-β-D-glucose en présence de NaOH (schéma 1). Les complexes d'or(I) **41** et **42** ont été synthétisés via la voie de transmétallation argentique.

### I.3. Protocole détaillé des synthèses.

### A. Protocole général pour la synthèse des fluoroarylimidazoles 1-5

Dans un ballon sous pression, deux équivalents d'imidazole, un équivalent de bromure de fluoraryle, un équivalent de K₂CO₃ et une quantité catalytique de CuSO₄ ont été mélangés à 205°C pendant 72 h. Après refroidissement à température ambiante, le produit brut a été extrait avec du MeOH et filtré à travers un tampon de célite pour donner le solide souhaité.

*1-(4-(Trifluorométhoxy)phényl)-1H-imidazole* **(1).** À partir d'imidazole (2,36 g, 34,73 mmol), 4-(trifluorométhoxy)iodobenzène (5 g, 17,36 mmol) et K₂CO₃ (2,4 g, 17,36 mmol). Solide jaune (2,69 g, 68%). ¹H NMR (400 MHz, CDCl₃): *δ* 7.66 (t, *J* = 1.1 Hz, 1H), 7.27-7.19 (m, 2H), 7.17-7.11 (m, 2H), 7.08 (t, *J* = 1.4 Hz, 1H), 7.01 (t, *J* = 1.1 Hz, 1H). ¹³C NMR (101 MHz, CDCl₃): *δ* 147.70 (1C), 135.71 (1C), 135.34 (1C), 130.51 (1C), 122.46 (2C), 122.24 (2C), 120.54 (1C), 117.97 (1C). ¹⁹F NMR (376 MHz, CDCl₃): *δ* -58.42 (3F). HMRS (ES⁺): Calc. pour C₁₆H₃F₃N₂O 229,0591, trouvé 229,0589.

*1-(3,5-Bis(trifluorométhyl)phényl)-1H-imidazole* **(2).** À partir d'imidazole (2,32 g, 34,12 mmol), 1,3-bis(trifluorométhyl)-5-bromobenzène (5 g, 17,06 mmol) et K₂CO₃ (2,36 g, 17,06 mmol). Solide blanc (3,87 g, 81%). ¹H NMR (400 MHz, CDCl₃): *δ* 7.95 (t, *J* = 1.1 Hz, 1H), 7.89-7.81 (m, 3H), 7.37 (t, *J* = 1.4 Hz, 1H), 7.23 (t, *J* = 1.1 Hz, 1H). ¹³C NMR (101 MHz, CDCl₃): *δ* 138.58 (1C), 135.42 (1C), 133.57 (2C), 131.58 (2C), 122.57 (2C), 121.32 (1C), 120.81 (1C), 117.83 (1C). ¹⁹F NMR (376 MHz, CDCl₃): *δ* -63.27 (6F). HMRS (ES⁺): Calc. pour C₁₁H₇F₆N₂ 281,0512, trouvé 281,0513.

*1-(3-(Trifluorométhoxy)phényl)-1H-imidazole* **(3).** À partir d'imidazole (1,42 g, 20,82 mmol), 3-(trifluorométhoxy)iodobenzène (3 g, 10,41 mol) et K₂CO₃ (1,44 g, 10,41 mol). Solide jaune (1,71 g, 72%). ¹H NMR (400 MHz, CDCl₃): *δ* 7.70 (t, *J* = 1.1 Hz, 1H), 7.30 (t, *J* = 8.2 Hz, 1H), 7.16-7.08 (m, 3H), 7.03-6.99 (m, 2H). ¹³C NMR (101 MHz, CDCl₃): *δ* 149.77 (1C), 138.38 (1C), 135.23 (1C), 130.97 (1C), 130.62 (1C), 120.18 (1C), 119.15 (1C), 119.07 (1C), 117.72 (1C), 113.81 (1C). ¹⁹F NMR (376 MHz, CDCl₃): *δ* -58.23 (3F). HMRS (ES⁺): Calc. pour C₁₆H₈F₃N₂O 229,0591, trouvé 229,0589.

### B. Protocole général pour la synthèse des sels de benzylimidazolium 6-10

Sous atmosphère d'azote, un mélange d'un équivalent d'un fluoroarylimidazole (300 mg) et de 1,1 équivalent de chlorure de benzyle dans du CH₃CN sec (10 mL) a été agité à 80°C pendant 3 jours. La solution a ensuite été évaporée, le solide résultant a été lavé avec de l'éther diéthylique et séché sous vide pour donner le solide souhaité.

*Chlorure de 3-Benzyl-1-(4-(trifluorométhoxy)phényl)-1H-imidazol-3-ium* (6). À partir de **1** (300 mg, 1,31 mmol) et de chlorure de benzyle (0,17 mL, 1,44 mmol). Solide blanc (409 mg, 88% de rendement). ¹H NMR (400 MHz, DMSO-*d*₆): *δ* 10.54 (pseudot, *J* = 1.6 Hz, 1H), 8.44 (t, *J* = 1.9 Hz, 1H), 8.15 (t, *J* = 1.8 Hz, 1H), 8.08-7.99 (m, 2H), 7.75-7.65 (m, 2H), 7.64-7.55 (m, 2H), 7.48-7.35 (m, 3H), 5.59 (s, 2H). ¹³C NMR (101 MHz, DMSO-*d*₆): *δ* 149.03 (1C), 136.66 (1C), 135.01 (1C), 134.19 (1C), 129.37 (2C), 129.26 (1C), 129.11 (2C), 124.71 (2C), 123.69 (1C), 123.19 (2C), 122.21 (1C), 120.04 (1C), 52.70 (1C). ¹⁹F NMR (376 MHz, DMSO-*d*₆): *δ* -57.00 (3F). HMRS (ES⁺): Calc. pour C₁₇H₁₄F₃N₂O, 319,1063, trouvé 319,1058.

*Chlorure de 3-Benzyl-1-(3,5-bis(trifluoromethyl)phenyl)-1H-imidazol-3-ium* **(7).** À partir de **2** (300 mg, 1,07 mmol) et de chlorure de benzyle (0,13 mL, 1,17 mmol). Solide blanc (280 mg, 64% de rendement). ¹H NMR (400 MHz, CDCl₃): *δ* 11.90 (pseudot, *J* = 1.6 Hz, 1H), 8.68 (t, *J* = 2.0 Hz, 1H), 8.57 (s, 2H), 7.88 (t, *J* = 1.8 Hz, 1H), 7.78 (s, 1H), 7.57-7.47 (m, 2H), 7.24-7.12 (m, 3H), 5.65 (s, 2H). ¹³C NMR (101 MHz, CDCl₃): *δ* 136.74 (1C), 135.88 (1C), 133.69 (2C), 132.84 (1C), 129.40 (1C), 129.19 (2C), 129.05 (2C), 123.68 (1C), 123.27 (1C), 122.55 (2C), 122.18 (2C), 121.81 (1C), 53.51 (1C). ¹⁹F NMR: (376 MHz, CDCl₃): *δ* -62.87 (6F). HMRS (ES⁺): Calc. pour C₁₃H₁₃F₆N₂ 371,0992, trouvé 371,0983.

*Chlorure de 3-Benzyl-1-(3-(trifluoromethoxy)phenyl)-1H-imidazol-3-ium chloride* **(8).** À partir de **3** (300 mg, 1,31 mmol) et de chlorure de benzyle (0,17 mL, 1,44 mmol). Solide blanc (380 mg, 82% de rendement). ¹H NMR (400 MHz, DMSO-*d*₆): *δ* 10.73 (pseudot, *J* = 1.6 Hz, 1H), 8.52 (t, *J* = 1.9 Hz, 1H), 8.20 (t, *J* = 1.8 Hz, 1H), 8.05 (d, *J* = 2.4 Hz, 1H), 7.99 (ddd, *J* = 8.3, 2.2, 0.8 Hz, 1H), 7.80 (t, *J* = 8.3 Hz, 1H), 7.66-7.56 (m, 3H), 7.47-7.34 (m, 3H), 5.61 (s, 2H). ¹³C NMR (101 MHz, DMSO-*d*₆): *δ* 149.26 (1C), 136.84 (1C), 136.52 (1C), 135.01 (1C), 132.48 (1C), 129.33 (2C), 129.24 (1C), 129.17 (2C), 123.71 (1C), 122.35 (1C), 121.96 (1C), 121.42 (1C), 120.40 (1C), 115.72 (1C), 52.67 (1C). ¹⁹F NMR (376 MHz, DMSO-*d*₆): *δ* -56.90 (3F). HMRS (ES⁺): Calc. pour C₁₇H₁₄F₃N₂O 319,1063, trouvé 319,1058.

*Chlorure de 3-Benzyl-1-(4-fluorophenyl)-1H-imidazol-3-ium chloride (9).* À partir de 1-(4-fluorophényl)-1*H*-imidazole **(4)** (303 mg, 1,87 mmol) et de chlorure de benzyle (0,24 mL, 2,05 mmol). Solide blanc (280 mg, 52% de rendement). ¹H NMR (400 MHz, DMSO-*d*₆): *δ* 10.56 (pseudot, *J* = 1.9 Hz, 1H), 8.40 (t, *J* = 1.9 Hz, 1H), 8.16 (t, *J* = 1.8 Hz, 1H), 8.00-7.90 (m, 2H), 7.64-7.57 (m, 2H), 7.58-7.47 (m, 2H), 7.45-7.37 (m, 3H), 5.59 (s, 2H). ¹³C NMR (101 MHz, DMSO-*d*₆): *δ* 169.21 (1C), 162.27 (1C), 136.79 (1C), 135.82 (1C), 129.28 (2C), 128.68 (1C), 128.21 (2C), 127.86 (2C), 123.76 (1C), 123.00 (1C), 116.17 (2C), 52.60 (1C). ¹⁹F NMR (376 MHz, DMSO-*d*₆): *δ* -111.65 (1F). HMRS (ES⁺): Calc. pour C₁₆H₁₄FN₂ 253,1147, trouvé 253,1141.

*Chlorure de 3-Benzyl-1-(4-(trifluoromethyl)phenyl)-1H-imidazol-3-ium chloride* **(10).** À partir de 1-(4-(trifluorométhyl)phényl)-1*H*-imidazole **(5)** (500 mg, 2,35 mmol) et de chlorure de benzyle (3,03 mL, 2,59 mmol). Solide blanc (709 mg, 89% de rendement). ¹H NMR (400 MHz, CDCl₃): *δ* 11.53 (pseudot, *J* = 1.6 Hz, 1H), 8.29 (t, *J* = 2.0 Hz, 1H), 7.96 (d, *J* = 8.4 Hz, 2H), 7.67 (t, *J* = 1.8 Hz, 1H), 7.50-7.41 (m, 4H), 7.11-7.04 (m, 3H), 5.57 (s, 2H). ¹³C NMR (101 MHz, CDCl₃): *δ* 136.87 (1C), 135.97 (1C), 132.91 (1C), 131.52 (1C), 129.35 (1C), 129.14 (2C), 128.97 (2C), 127.40 (2C), 123.24 (1C), 122.95 (1C), 121.82 (2C), 121.17 (1C), 53.23 (1C). ¹⁹F NMR (376 MHz, CDCl₃): *δ* -63.04 (3F). HMRS (ES⁺): Calc. pour C₁₇H₁₄F₃N₂ 303,1112, trouvé 303,1109.

### C. Protocole général pour la synthèse des sels d'isopropylimidazolium 11-15

Sous atmosphère d'azote, un équivalent d'un fluoroarylimidazole a été dissous dans du 2-bromopropane (3 mL). Le mélange a été agité à 85°C pendant 2 jours. La solution a ensuite été évaporée, le solide résultant a été lavé avec de l'éther diéthylique et séché sous vide pour donner le solide souhaité.

Bromure de *3-Isopropyl-1-(4-(trifluorométhoxy)phényl)-1H-imidazol-3-ium* **(11).** À partir de **1** (318 mg, 1,39 mmol). Solide blanc (217 mg, 45% de rendement). ¹H NMR (400 MHz, CDCl₃): *δ* 10.89 (pseudot, *J* = 1.7 Hz, 1H), 8.11 (t, *J* = 1.9 Hz, 1H), 8.08-7.93 (m, 2H), 7.83 (t, *J* = 1.9 Hz, 1H), 7.33-7.23 (m, 2H), 5.04 (hept, *J* = 6.7 Hz, 1H), 1.60 (d, *J* = 6.7 Hz, 6H). ¹³C NMR (101 MHz, CDCl₃): *δ* 149.70 (1C), 134.53 (1C), 132.70 (1C), 123.78 (2C), 122.48 (2C), 121.53 (1C), 121.32 (1C), 120.10 (1C), 54.01 (1C), 23.02 (2C). ¹⁹F NMR (376 MHz, CDCl₃): *δ* -57.96 (3F). HMRS (ES⁺): Calc. pour C₁₃H₁₄F₃N₂O 271.1064, trouvé 271,1058.

Bromure de *1-(3,5-Bis(trifluorométhyl)phényl)-3-isopropyl-1H-imidazol-3-ium* **(12).** À partir de **2** (300 mg, 1,07 mmol). Solide blanc (82 mg, 19% de rendement). ¹H NMR (400 MHz, CDCl₃): *δ* 11.36 (pseudot, *J* = 1.7 Hz, 1H), 8.68-8.58 (m, 2H), 8.43 (t, *J* = 2.0 Hz, 1H), 8.05 (t, *J* = 1.9 Hz, 1H), 7.97-7.89 (m, 1H), 5.12 (hept, *J* = 6.7 Hz, 1H), 1.71 (d, *J* = 6.7 Hz, 6H). ¹³C NMR (101 MHz, CDCl₃): *δ* 135.89 (1C), 135.56 (1C), 133.79 (2C), 123.61 (1C), 123.15 (2C), 122.21 (1C), 122.04 (2C), 121.90 (1C), 54.57 (1C), 22.98 (2C). ¹⁹F NMR (376 MHz, CDCl₃): *δ* -62.70 (6F). HMRS (ES⁺): Calc. pour C₁₄H₁₃F₆N₂ 323.0981, trouvé 323,0983.

Bromure de *3-Isopropyl-1-(3-(trifluorométhoxy)phényl)-1H-imidazol-3-ium* **(13).** À partir de **3** (300 mg, 1,31 mmol). Solide blanc (460 mg, 100% de rendement). ¹H NMR (400 MHz, CDCl₃): *δ* 10.86 (pseudot, *J* = 1.7 Hz, 1H), 8.02 (t, *J* = 2.0 Hz, 1H), 7.90 (t, *J* = 1.9 Hz, 1H), 7.85 (ddd, *J* = 8.3, 2.3, 0.8 Hz, 1H), 7.65 (d, *J* = 2.3 Hz, 1H), 7.42 (d, *J* = 8.3 Hz, 1H), 7.14 (ddd, *J* = 8.3, 2.2, 1.1 Hz, 1H), 5.01 (hept, *J* = 6.7 Hz, 1H), 1.52 (d, *J* = 6.7 Hz, 6H). ¹³C NMR (101 MHz, CDCl₃): *δ* 149.69 (1C), 135.48 (1C), 134.62 (1C), 131.84 (1C), 121.85 (1C), 121.74 (1C), 121.33 (1C), 120.70 (1C), 120.57 (1C), 114.86 (1C), 54.02 (1C), 22.92 (2C). ¹⁹F NMR (376 MHz, CDCl₃): *δ* -58.00 (3F). HMRS (ES⁺): Calc. pour C₁₃H₁₄F₃N₂O 271,1063, trouvé 271,1058.

Bromure de *1-(4-Fluorophényl)-3-isopropyl-1H-imidazol-3-ium* **(14).** À partir de **4** (300 mg, 1,85 mmol). Solide blanc (438 mg, 83% de rendement). ¹H NMR (400 MHz, CDCl₃): *δ* 10.72 (pseudot, *J* = 1.7 Hz, 1H), 7.96 (t, *J* = 2.0 Hz, 1H), 7.87-7.81 (m, 2H), 7.79 (t, *J* = 1.9 Hz, 1H), 7.11-6.99 (m, 2H), 4.99 (hept, *J* = 6.7 Hz, 1H), 1.54 (d, *J* = 6.8 Hz, 6H). ¹³C NMR (101 MHz, CDCl₃): *δ* 162.76 (1C), 134.35 (1C), 130.56 (1C), 124.22 (2C), 121.57 (1C), 121.23 (1C), 117.21 (2C), 53.84 (1C), 23.05 (2C). ¹⁹F NMR (376 MHz, CDCl₃): *δ* -110.07 (1F). HMRS (ES⁺): Calc. pour C₁₂H₁₄FN₂ 205,1145, trouvé 205,1141.

Bromure de *3-Isopropyl-1-(4-(trifluoromethyl)phenyl)-1H-imidazol-3-ium* **(15).** À partir de **5** (500 mg, 2,36 mmol). Solide blanc (490 mg, 62% de rendement). ¹H NMR (400 MHz, DMSO-*d*₆): *δ* 10.95 (pseudot, *J* = 1.7 Hz, 1H), 8.22 (t, *J* = 2.0 Hz, 1H), 8.17-8.08 (m, 2H), 7.83 (t, *J* = 1.9 Hz, 1H), 7.70-7.56 (m, 2H), 5.00 (hept, *J* = 6.7 Hz, 1H), 1.57 (d, *J* = 6.7 Hz, 6H), 1.56. ¹³C NMR (101 MHz, CDCl₃): *δ* 138.36 (1C), 134.65 (1C), 131.66 (1C), 127.46 (2C), 123.09 (1C), 122.42 (2C), 121.73 (1C), 121.48 (1C), 54.14 (1C), 22.99 (2C). ¹⁹F NMR (376 MHz, CDCl₃): *δ* -62.98 (3F). HMRS (ES⁺): Calc. pour C₁₃H₁₄F₃N₂ 255,1116, trouvé 255,1109.

### D. Protocole général pour la synthèse des sels de quinolylimidazolium 16-20

Dans un ballon sous pression, un équivalent de fluoroarylimidazole et 1,05 équivalent de 2-chloroquinoléine ont été mélangés à 170°C pendant 18 h. Après refroidissement à température ambiante, le produit brut a été dissous dans du CH₂Cl₂ et précipité avec de l'éther diéthylique, le solide résultant a été filtré et séché sous vide pour donner le solide souhaité.

*Chlorure de 3-(Quinolin-2-yl)-1-(4-(trifluoromethoxy)phenyl)-1H-imidazol-3-ium* **(16).** À partir de **1** (216 mg, 0,95 mmol) et 2-chloroquinoléine (162 mg, 0,99 mmol). Solide beige (251 mg, 67% de rendement). ¹H NMR (300 MHz, DMSO-*d*₆): *δ* ¹H NMR 11.19 (pseudot, *J* = 1.7 Hz, 1H), 8.95 (t, *J* = 1.9 Hz, 1H), 8.88 (d, *J* = 8.8 Hz, 1H), 8.72 (t, *J* = 2.0 Hz, 1H), 8.55 (d, *J* = 8.8 Hz, 1H), 8.34-8.22 (m, 2H), 8.20-8.10 (m, 2H), 7.95 (ddd, *J* = 8.4, 6.8, 1.5 Hz, 1H), 7.85-7.59 (m, 3H). ¹³C NMR (75 MHz, DMSO-*d*₆): *δ* 149.39 (1C), 145.92 (1C), 145.63 (1C), 141.74 (1C), 136.00 (1C), 134.01 (1C), 132.22 (1C), 128.82 (1C), 128.76 (1C), 128.54 (1C), 128.35 (1C), 125.16 (2C), 123.16 (2C), 122.89 (1C), 120.43 (1C), 120.40 (1C), 113.57 (1C). ¹⁹F NMR (282 MHz, DMSO-*d*₆) *δ* -56.93 (3F). HMRS (ES⁺): Calc. pour C₁₉H₁₃F₃N₃O 356,1007, trouvé 356,1011.

*Chlorure de 1-(3,5-Bis(trifluoromethyl)phenyl)-3-(quinolin-2-yl)-1H-imidazol-3-ium* **(17).** À partir de **2** (600 mg, 2,13 mmol) et 2-chloroquinoléine (367 mg, 2,24 mmol). Solide beige (396 mg, 42% de rendement). ¹H NMR (400 MHz, DMSO-*d*₆): *δ* 11.35 (pseudot, *J* = 1.9 Hz, 1H), 9.01 (t, *J* = 1.9 Hz, 1H), 8.93-8.91 (m, 4H), 8.5-8.48 (m, 1H), 8.46 (s, 1H), 8.21 (dd, *J* = 8.2, 1.4 Hz, 1H), 8.14 (d, *J* = 8.5 Hz, 1H), 7.98 (ddd, *J* = 8.4, 6.9, 1.4 Hz, 1H), 7.80 (ddd, *J* = 8.1, 6.8, 1.2 Hz, 1H). ¹³C NMR (101 MHz, DMSO-d₆): *δ* 145.97 (1C), 145.53 (1C), 141.92 (1C), 136.83 (1C), 136.65 (1C), 132.35 (1C), 132.26 (2C), 128.88 (1C), 128.76 (1C), 128.68 (1C), 128.44 (1C), 124.50 (2C), 124.21 (1C), 123.08 (1C), 123.01 (2C), 120.44 (1C), 113.48 (1C). ¹⁹F NMR (376 MHz, DMSO-*d₆*) *δ* -61.12 (6F). HMRS (ES⁺): Calc. pour C₂₀H₁₂F₆N₃ 408.0932, trouvé 408.0935.

*Chlorure de 3-(Quinolin-2-yl)-1-(3-(trifluoromethoxy)phenyl)-1H-imidazol-3-ium* **(18).** À partir de **3** (216 mg, 0,95 mmol) et 2-chloroquinoléine (163 mg, 0,10 mmol). Solide blanc (206 mg, 53% de rendement). ¹H NMR (300 MHz, DMSO-*d*₆): *δ* 11.17 (pseudot, *J* = 1.9 Hz, 1H), 8.96 (t, *J* = 1.9 Hz, 1H), 8.90 (d, *J* = 8.8 Hz, 1H), 8.75 (t, *J* = 1.8 Hz, 1H), 8.52 (d, *J* = 8.8 Hz, 1H), 8.27-8.12 (m, 4H), 8.00-7.67 (m, 4H). ¹³C NMR (101 MHz, DMSO-*d₆*): *δ* 149.28 (1C), 145.94 (1C), 145.61 (1C), 141.78 (1C), 136.34 (1C), 136.09 (1C), 132.53 (1C), 132.27 (1C), 128.83 (1C), 128.77 (1C), 128.59 (1C), 128.38 (1C), 122.91 (1C), 122.69 (1C), 121.90 (1C), 120.46 (1C), 120.45 (1C), 1116.21 (1C), 113.56 (1C). ¹⁹F NMR (376 MHz, DMSO-*d₆*): *δ* -56.81 (3F). HMRS (ES⁺): Calc. pour C₁₉H₁₃F₃N₃O 356,1016, trouvé 356,1011.

*Chlorure de 1-(4-Fluorophenyl)-3-(quinolin-2-yl)-1H-imidazol-3-ium* **(19).** À partir de **4** (200 mg, 1,23 mmol) et 2-chloroquinoléine (208 mg, 1,27 mmol). Solide beige (165 mg, 42% de rendement). ¹H NMR (300 MHz, DMSO-*d*₆): *δ* 10.87 (pseudot, *J* = 1.6 Hz, 1H), 8.92-8.88 (m, 2H), 8.62 (t, *J* = 1.9 Hz, 1H), 8.38 (d, *J* = 8.8 Hz, 1H), 8.21-8.06 (m, 4H), 7.97 (ddd, *J* = 8.5, 6.9, 1.5 Hz, 1H), 7.79 (ddd, *J* = 8.1, 7.0, 1.2 Hz, 1H). ¹³C NMR (101 MHz, DMSO-*d₆*): *δ* 163.00 (1C), 145.97 (1C), 145.66 (1C), 141.80 (1C), 135.67 (1C), 132.30 (1C), 131.59 (1C), 128.83 (1C), 128.77 (1C), 128.60 (1C), 128.34 (1C), 125.43 (2C), 123.08 (1C), 120.38 (1C), 117.51 (2C), 113.40 (1C). ¹⁹F NMR (376 MHz, DMSO-*d₆*): *δ* - 110.85 (1F). HMRS (ES⁺): Calc. pour C₁₈H₁₃FN₃ 290,1093, trouvé 290,1094.

*Chlorure de 3-Quinolyl-1-(4-(trifluoromethyl)phenyl)-1H-imidazol-3-ium* **(20).** À partir de **5** (200 mg, 0,94 mmol) et 2-chloroquinoléine (162,0 mg, 0,99 mmol). Solide beige (292 mg, 83% de rendement). ¹H NMR (400 MHz, DMSO-*d*₆): *δ* 11.31 (pseudot, *J* = 1.6 Hz, 1H), 8.97 (t, *J* = 1.9 Hz, 1H), 8.88 (d, *J* = 8.8 Hz, 1H), 8.81 (t, *J* = 1.9 Hz, 1H), 8.58 (d, *J* = 8.8 Hz, 1H), 8.38 (d, *J* = 8.3 Hz, 2H), 8.20-8.11 (m, 4H), 7.96 (ddd, *J* = 8.2, 6.9, 1.5 Hz, 1H), 7.78 (ddd, *J* = 8.1, 6.9, 1.2 Hz, 1H). ¹³C NMR (101 MHz, DMSO-*d₆*): *δ* 145.91 (1C), 145.60 (1C), 141.74 (1C), 138.12 (1C), 136.21 (1C), 132.23 (1C), 130.57 (1C), 128.82 (1C), 128.77 (1C), 128.57 (1C), 128.37 (1C), 127.76 (2C), 124.10 (1C), 123.63 (2C), 122.59 (1C), 120.57 (1C), 113.62 (1C). ¹⁹F NMR (376 MHz, DMSO-*d₆*): *δ* -61.13 (3F). HMRS (ES⁺): Calc. pour C₁₉H₁₃F₃N₃ 340,1063, trouvé 340,1062.

### E. Protocole général pour la synthèse des complexes d'or(I) 21-30

Sous atmosphère d'azote et protection de la lumière, un équivalent d'un sel d'imidazolium et 0,48 équivalent d'Ag₂O ont été dissous dans du CH₂Cl₂ sec et agités à température ambiante pendant 18 h. 1,07 équivalent d'Au(SMe₂)Cl a ensuite été ajouté et le mélange résultant a été agité pendant 2 h. Après filtration à travers un tampon de célite, le solvant a été éliminé sous pression et le solide a été séché sous vide pour donner le complexe souhaité sous forme d'un solide blanc.

**Complexe 21.** À partir de **6** (100 mg, 0,28 mmol), Ag₂O (30 mg, 0,13 mmol) and Au(SMe₂) (88 mg, 0,30 mmol). Solide blanc (148 mg, 96% de rendement). ¹H NMR (300 MHz, DMSO-*d*₆): *δ* 7.94-7.89 (m, 3H), 7.83 (d, *J* = 2.0 Hz, 1H), 7.65-7.57 (m, 2H), 7.51-7.32 (m, 5H), 5.49 (s, 2H). ¹³C NMR (101 MHz, DMSO-*d₆*): *δ* 169.23 (1C), 148.62 (1C), 138.28 (1C), 136.73 (1C), 129.28 (2C), 128.70 (1C), 128.22 (2C), 127.66 (2C), 123.67 (1C), 123.21 (1C), 122.54 (2C), 120.43 (1C), 54.67 (1C). ¹⁹F NMR (376 MHz, DMSO-*d₆*) *δ* - 56.85 (3F). HMRS (DCl-CH₃⁺): Calc. pour C₁₇H₁₃AuF₃N₂O 515,0635, trouvé 515,0646.

**Complexe 22.** À partir de **7** (118 mg, 0,29 mmol), Ag₂O (33 mg, 0,14 mmol) et Au(SMe₂)Cl (91 mg, 0,31 mmol). Solide blanc (170 mg, 97% de rendement). ¹H NMR (300 MHz, DMSO-*d*₆): *δ* 8.61 (s, 2H), 8.38-8.29 (m, 1H), 8.07 (d, *J* = 2.1 Hz, 1H), 7.86 (d, *J* = 2.1 Hz, 1H), 7.53-7.32 (m, 5H), 5.50 (s, 2H). ¹³C NMR (101 MHz, DMSO-*d₆*): *δ* 170.07 (1C), 140.82 (1C), 136.57 (1C), 131.60 (2C), 129.25 (2C), 128.71 (1C), 128.24 (2C), 127.07 (2C), 123.75 (1C), 123.41 (1C), 123.22 (1C), 123.20 (2C), 54.74 (1C). ¹⁹F NMR (376 MHz, DMSO-*d₆*): *δ* -61.26 (6F). HMRS (DCl-CH₄⁺): Calc. pour C₁₈H₁₂AuF₆N₂ 567,0568, trouvé 567,0570.

**Complexe 23.** À partir de **8** (103 mg, 0,29 mmol), Ag₂O (33 mg, 0,14 mmol) et (91 mg, 0,31 mmol). Solide blanc (150 mg, 94% de rendement). ¹H NMR (400 MHz, DMSO-*d*₆): *δ* 7.94-7.81 (m, 4H), 7.73 (dd, *J* = 8.2 Hz, 1H), 7.58 (ddd, *J* = 8.3, 2.3, 1.1 Hz, 1H), 7.50-7.33 (m, 5H), 5.49 (s, 2H). ¹³C NMR (101 MHz, DMSO-*d₆*): *δ* 169.39 (1C), 148.78 (1C), 140.63 (1C), 136.68 (1C), 131.79 (1C), 129.28 (2C), 128.71 (1C), 128.25 (2C), 124.71 (1C), 123.57 (1C), 123.26 (1C), 121.89 (1C), 120.46 (1C), 118.74 (1C), 54.73 (1C). ¹⁹F NMR (376 MHz, DMSO-*d₆*): *δ* -56.77 (3F). HMRS (DCl-CH₄⁺): Calc. pour C₁₇H₁₃AuF₃N₂O 515,0657, trouvé 515,0646.

**Complexe 24.** À partir de **9** (100 mg, 0,35 mmol), Ag₂O (39 mg, 0,17 mmol) et Au(SMe₂)Cl (112 mg, 0,38 mmol). Solide blanc (146 mg, 86% de rendement). ¹H NMR (400 MHz, DMSO-*d*₆): *δ* 7.84 (d, *J* = 2.0 Hz, 1H), 7.82-7.77 (m, 3H), 7.47-7.34 (m, 7H), 5.47 (s, 2H). ¹³C NMR (101 MHz, DMSO-*d₆*): *δ* 169.21 (1C), 162.28 (1C), 136.79 (1C), 135.83 (1C), 129.28 (2C), 128.68 (1C), 128.21 (2C), 127.87 (2C), 123.76 (1C), 123.00 (1C), 116.77 (2C), 54.59 (1C). ¹⁹F NMR (376 MHz, DMSO) *δ* -112.59 (1F). HMRS (ES⁺): Calc. pour C₁₈H₁₆AuFN₃ (M⁺ + CH₃CN) 490,1000, trouvé 490,0994.

**Complexe 25.** À partir de **10** (100 mg, 0,30 mmol), Ag₂O (33 mg, 0,14 mmol) et Au(SMe₂)Cl (95 mg, 0,32 mmol). Solide blanc (135 mg, 84% de rendement). ¹H NMR (300 MHz, DMSO-*d*₆): *δ* 8.05-7.97 (m, 4H), 7.95 (d, *J* = 2.0 Hz, 1H), 7.87 (d, *J* = 2.1 Hz, 1H), 7.54-7.27 (m, 5H), 5.50 (s, 2H). ¹³C NMR (75 MHz, DMSO-*d₆*): *δ* 169.32 (1C), 142.52 (1C), 136.68 (1C), 129.79 (1C), 129.30 (2C), 128.73 (1C), 128.25 (2C), 127.18 (2C), 126.44 (2C), 124.27 (1C), 123.48 (2C), 54.79 (1C). ¹⁹F NMR (282 MHz, DMSO-*d₆*): *δ* -61.03 (3F). HMRS (ES⁺): Calc. pour C₁₉H₁₆AuF₃N₃ (M⁺ + CH₃CN) 540,0962, trouvé 540,0963.

**Complexe 26.** À partir de **11** (100 mg, 0,29 mmol), Ag₂O (33 mg, 0,14 mmol) et Au(SMe₂)Cl (91 mg, 0,31 mmol). Solide blanc (134 mg, 92% de rendement). ¹H NMR (400 MHz, DMSO-*d*₆): *δ* 7.93-7.85 (m, 4H), 7.64-7.58 (m, 2H), 4.99 (hept, *J* = 6.8 Hz 1H), 1.52 (dd, *J* = 6.8, 1.5 Hz, 6H). ¹³C NMR (75 MHz, DMSO-*d₆*): *δ* 167.60 (1C), 148.54 (1C), 138.49 (1C), 127.59 (2C), 123.59 (1C), 122.53 (2C), 120.44 (1C), 119.59 (1C), 54.41 (1C), 23.16 (2C). ¹⁹F NMR (282 MHz, DMSO-*d₆*): *δ* -56.84 (3F). HMRS (ES⁺): Calc. For C₁₃H₁₃Au_{F}3N₂O 467.07, found 467.06. HMRS (DCl-CH₄⁺): Calc. pour C₁₃H₁₃AuF₃N₂O 467,0647 trouvé 467,0646.

**Complexe 27.** À partir de **12** (60 mg, 0,15 mmol), Ag₂O (16 mg, 0,07 mmol) et Au(SMe₂)Cl (47 mg, 0,16 mmol). Solide blanc (83 mg, 100% de rendement). ¹H NMR (300 MHz, DMSO-*d*₆): *δ* 8.58 (s, 2H), 8.33 (s, 1H), 8.06 (d, *J* = 2.1 Hz, 1H), 7.96 (d, *J* = 2.1 Hz, 1H), 5.01 (hept, *J* = 6.7 Hz, 1H), 1.52 (d, *J* = 6.7 Hz, 6H). ¹³C NMR (75 MHz, DMSO-*d₆*): *δ* 168.42 (1C), 141.01 (1C), 131.61 (2C), 126.92 (2C), 123.62 (1C), 123.19 (2C), 123.10 (1C), 119.86 (1C), 54.59 (1C), 23.09 (2C). ¹⁹F NMR (376 MHz, DMSO-*d₆*): *δ* -61.30 (6F). HMRS (DCl-CH₄⁺): Calc. pour C₁₄H₁₂AuF₆N₂ 519,0544 trouvé, 519,0570.

**Complexe 28.** À partir de **13** (101 mg, 0.29 mmol) Ag₂O (33 mg, 0.14 mmol) and Au(SMe₂)Cl (91 mg, 0.31 mmol). White solid (143 mg, 98% de rendement). ¹H NMR (400 MHz, DMSO-*d*₆): *δ* 7.92-7.90 (m, 2H), 7.88-7.78 (m, 2H), 7.72 (dd, *J* = 8.1 Hz, 1H), 7.57 (ddd, *J* = 8.3, 2.2, 1.1 Hz, 1H), 4.99 (hept, *J* = 6.7 Hz, 1H), 1.52 (d, *J* = 6.7 Hz, 6H). ¹³C NMR (101 MHz, DMSO-*d₆*): *δ* 167.75 (1C), 148.78 (1C), 140.83 (1C), 131.79 (1C), 124.62 (1C), 123.48 (1C), 121.79 (1C), 120.46 (1C), 119.66 (1C), 118.63 (1C), 54.49 (1C), 23.13 (2C). ¹⁹F NMR (376 MHz, DMSO-*d₆*): *δ* -56.77 (3F). HMRS (DCl-CH₄⁺): Calc. pour C₁₃H₁₃AuF₃N₂O 467,0646, trouvé 467,0646.

**Complexe 29.** À partir de **14** (103 mg, 0,36 mmol) Ag₂O (39 mg, 0,17 mmol) et Au(SMe₂)Cl (115 mg, 0,39 mmol). Solide blanc (157 mg, 100% de rendement). ¹H NMR (400 MHz, DMSO-*d*₆): *δ* 7.88 (d, *J* = 2.1 Hz, 1H), 7.82 (d, *J* = 2.1 Hz, 1H), 7.81-7.73 (m, 2H), 7.49-7.38 (m, 2H), 4.98 (hept, *J* = 6.7 Hz, 1H), 1.52 (d, *J* = 6.7 Hz, 6H). ¹³C NMR (101 MHz, DMSO-*d₆*): *δ* 167.59 (1C), 162.21 (1C), 136.04 (1C), 127.79 (2C), 123.66 (1C), 119.37 (1C), 116.76 (2C), 54.28 (1C), 23.18 (2C). ¹⁹F NMR (376 MHz, DMSO-*d₆*) *δ* -112.77 (1F). HMRS (DCl-CH₄⁺): Calc. pour C₁₂H₁₃AuFN₂ 401,0726, trouvé 401,0728.

**Complexe 30.** À partir de **15** (100 mg, 0,30 mmol) Ag₂O (33 mg, 0,14 mmol) et Au(SMe₂)Cl (94 mg, 0,32 mmol). Solide blanc (141 mg, 97% de rendement). ¹H NMR (300 MHz, DMSO-*d*₆): *δ* 8.07-7.86 (m, 6H), 5.00 (hept, *J* = 6.7 Hz, 1H), 1.53 (d, *J* = 6.7 Hz, 6H). ¹³C NMR (101 MHz, DMSO-*d₆*): *δ* 167.66 (1C), 142.72 (1C), 129.56 (1C), 127.16 (2C), 126.36 (2C), 124.27 (1C), 123.40 (1C), 119.87 (1C), 54.56 (1C), 23.14 (2C). ¹⁹F NMR (282 MHz, DMSO-*d₆*): *δ* -61.02 (3F). HMRS (ES⁺): Calc. pour C₁₅H₁₆AuF₃N₃ (M⁺ + CH₃CN) 492,0964, trouvé 492,0962.

### F. Protocole général pour la synthèse des complexes d'or(I) 31-35

Sous atmosphère d'azote, un équivalent d'un sel d'imidazolium, 1,08 équivalent de Au(SMe₂)Cl et deux équivalents de K₂CO₃ ont été dissous dans 10 ml d'acétone et chauffés sous reflux pendant 18 h. Le solvant a été ensuite évaporé, le produit brut a été dissous dans du DCM (10 mL) et filtré à travers un tampon de silice. Le solvant a été concentré sous vide et l'ajoût de pentane a entraîné la précipitation d'un solide, qui a été séché sous vide pour donner le complexe souhaité sous forme d'un solide blanc.

**Complexe 31.** À partir de **16** (63 mg, 0,14 mmol), Au(SMe₂)Cl (50 mg, 0,17 mmol) et K₂CO₃ (44 mg, 0,32 mmol). Solide blanc (39 mg, 41% de rendement). ¹H NMR (300 MHz, CD₃CN): *δ* 8.63-8.47 (m, 2H), 8.15-8.03 (m, 3H), 7.98-7.85 (m, 3H), 7.73 (ddd, *J* = 8.2, 6.9, 1.2 Hz, 1H), 7.67 (d, *J* = 2.1 Hz, 1H), 7.57-7.53 (m, 2H). ¹³C NMR (101 MHz, CD₃CN): *δ* 169.80 (1C), 149.36 (1C), 149.14 (1C), 146.38 (1C), 139.59 (1C), 138.26 (1C), 131.11 (1C), 128.64 (1C), 128.08 (1C), 127.92 (1C), 127.66 (1C), 127.51 (2C), 123.08 (1C), 121.92 (2C), 121.49 (1C), 117.56 (1C), 116.17 (1C). ¹⁹F NMR (376 MHz, CD₃CN): δ - 58.66 (3F). HMRS (DCI-CH₄⁺): Calc. pour C₁₉H₁₂AuF₃N₃O 552,0577, trouvé 552,0598.

**Complexe 32.** À partir de **17** (53 mg, 0,12 mmol), Au(SMe₂)Cl (38 mg, 0,13 mmol) et K₂CO₃ (33 mg, 0,24 mmol). Solide blanc (62 mg, 81% de rendement). ¹H NMR (300 MHz, DMSO-*d*₆): *δ* 8.79 (dd, *J* = 8.5, 1.5 Hz, 1H), 8.76-8.68 (s, 2H), 8.48-8.38 (m, 3H), 8.33 (d, *J* = 2.2 Hz, 1H), 8.19-8.09 (m, 2H), 7.94 (ddd, *J* = 8.5, 6.9, 1.5 Hz, 1H), 7.77 (ddd, *J* = 8.2, 6.9, 1.2 Hz, 1H). ¹³C NMR (101 MHz, DMSO-*d₆*): *δ* 170.16 (1C), 149.63 (1C), 146.34 (1C), 141.03 (1C), 140.58 (1C), 131.82 (1C), 131.66 (2C), 128.91 (1C), 128.69 (1C), 128.34 (1C), 128.20 (1C), 127.41 (2C), 124.20 (1C), 123.67 (1C), 123.22 (2C), 122.69 (1C), 116.97 (1C). ¹⁹F NMR (376 MHz, DMSO-d₆): *δ* -61.24 (6F). HMRS (DCI-CH₄⁺): Calc. pour C₂₆H₁₁AuF₆N₃ 604,0529, trouvé 604,0523.

**Complexe 33.** À partir de **18** (90 mg, 0,23 mmol), Au(SMe₂)Cl (74 mg, 0,25 mmol) et K₂CO₃ (64 mg, 0,46 mmol). Solide blanc (65 mg, 48% de rendement). ¹H NMR (300 MHz, DMSO-*d*₆): *δ* 8.76 (dd, *J* = 8.9, 1.2 Hz, 1H), 8.45-8.37 (m, 2H), 8.20-8.08 (m, 3H), 8.02-7.89 (m, 3H), 7.82-7.73 (m, 2H), 7.63 (ddd, J = 8.4, 2.3, 1.1 Hz, 1H). ¹³C NMR (101 MHz, DMSO-*d₆*): *δ* 169.42 (1C), 149.73 (1C), 148.80 (1C), 146.33 (1C), 140.82 (1C), 140.44 (1C), 131.88 (1C), 131.74 (1C), 128.91 (1C), 128.64 (1C), 128.26 (1C), 128.15 (1C), 124.99 (1C), 124.08 (1C), 122.59 (1C), 122.26 (1C), 120.50 (1C), 119.05 (1C), 117.07 (1C). ¹⁹F NMR (282 MHz, DMSO-*d₆*) *δ* -56.76 (3F). HMRS (DCI-CH₄⁺): Calc. pour C₁₉H₁₂AuF₃N₃O 552,0585, trouvé 552,0598.

**Complexe 34.** À partir de **19** (114 mg, 0,35 mmol), Au(SMe₂)Cl (112 mg, 0,38 mmol) et K₂CO₃ (97 mg, 0,70 mmol). Solide blanc (73 mg, 40% de rendement). ¹H NMR (300 MHz, DMSO-*d*₆): *δ* 8.76 (d, *J* = 8.7 Hz, 1H), 8.42-8.36 (m, 2H), 8.18-8.09 (m, 3H), 7.95-7.90 (m, 3H), 7.79-7.73 (m, 1H), 7.53-7.47 (m, 2H). ¹³C NMR (101 MHz, DMSO-*d₆*): *δ* 169.21 (1C), 162.51 (1C), 149.77 (1C), 146.34 (1C), 140.41 (1C), 136.05 (1C), 131.73 (1C), 128.90 (2C), 128.65 (1C), 128.23 (1C), 128.13 (2C), 124.30 (1C), 122.35 (1C), 117.03 (2C), 116.75 (1C). ¹⁹F NMR (282 MHz, DMSO-*d₆*): *δ* -112.05 (1F). HMRS (DCI-CH₄⁺): Calc. pour C₁₈H₁₂AuFN₃ 486,0682, trouvé 486,0681.

**Complexe 35.** À partir de **20** (71 mg, 0,19 mmol), Au(SMe₂)Cl (62 mg, 0,21 mmol) et K₂CO₃ (53 mg, 0,38 mmol). Solide blanc (51 mg, 47% de rendement). ¹H NMR (400 MHz, DMSO-*d*₆): *δ* 8.78 (d, *J* = 8.7 Hz, 1H), 8.48-8.37 (m, 2H), 8.23 (d, *J* = 2.2 Hz, 1H), 8.20-8.03 (m, 6H), 7.94 (ddd, *J* = 8.4, 6.9, 1.5 Hz, 1H), 7.77 (ddd, *J* = 8.1, 6.9, 1.2 Hz, 1H). ¹³C NMR (101 MHz, DMSO-*d₆*): *δ* 169.32 (1C), 149.73 (1C), 146.32 (1C), 142.74 (1C), 140.48 (1C), 131.77 (1C), 130.03 (1C), 128.91 (1C), 128.66 (1C), 128.30 (1C), 128.18 (1C), 127.26 (2C), 127.22 (2C), 124.27 (1C), 124.00 (1C), 122.81 (1C), 117.10 (1C). ¹⁹F NMR (376 MHz, DMSO-*d₆*): *δ* -61.03 (3F). HMRS (DCI-CH₄⁺): Calc. pour C₁₉H₁₂AuF₃N₃ 536,0626, trouvé 536,0649.

### G. Protocole général pour la synthèse des complexes d'or(I) 36-40

Sous atmosphère d'azote et à l'abri de la lumière, un tube de Schlenk a été chargé avec un équivalent de tétraacétate de thio-β-D-glucose dans 2 ml d'acétone dégazée. Un équivalent de NaOH 1 M a été ajouté et le mélange a été agité à température ambiante pendant 30 min. Après cela, le mélange réactionnel a été transféré sur une solution d'un complexe d'or(I) (0,9 équivalent) dans de l'acétone dégazée (2 ml) à 0°C. A la fin de l'addition, le bain de glace a été retiré et le mélange a été agité 18 h à température ambiante. Après évaporation du solvant, le produit brut a été dissous dans du CH₂Cl₂ et la solution a été filtrée sur un tampon de silice. Le solvant a été concentré sous vide et l'addition de pentane a conduit à la précipitation d'un solide, qui a été séché sous vide pour donner le complexe souhaité sous forme d'un solide blanc.

**Complexe 36**. À partir de tétraacétate de thio-β-D-glucose (36 mg, 0,1 mmol) et complexe **21** (50 mg, 0,09 mmol). Solide blanc (55 mg, 70%). ¹H NMR (300 MHz, CDCl₃): *δ* 7.91-7.73 (m, 2H), 7.52-7.32 (m, 7H), 7.20 (d, *J* = 2.0 Hz, 1H), 7.07 (d, *J* = 2.0 Hz, 1H), 5.72-5.45 (m, 2H), 5.12 (ddd, *J* = 9.3, 7.1, 2.1 Hz, 1H), 5.01-4.87 (m, 3H), 4.15 (dd, *J* = 12.2, 4.9 Hz, 1H), 4.03 (dd, *J* = 12.2, 2.5 Hz, 1H), 3.66 (ddd, *J* = 10.0, 4.9, 2.5 Hz, 1H), 2.05 (s, 3H), 2.01 (s, 3H), 1.99 (s, 3H), 1.96 (s, 3H). ¹³C NMR (101 MHz, CDCl₃): *δ* 182.69 (1C), 170.70 (1C), 170.26 (1C), 169.85 (1C), 169.44 (1C), 149.13 (1C), 137.50 (1C), 134.96 (1C), 129.17 (2C), 128.86 (1C), 128.18 (2C), 126.14 (2C), 122.09 (2C), 121.60 (1C), 121.11 (1C), 120.31 (1C), 83.01 (1C), 77.59 (1C), 75.63 (1C), 74.19 (1C), 68.98 (1C), 62.89 (1C), 55.28 (1C), 21.12 (1C), 20.73 (1C), 20.62 (1C), 20.60 (1C). ¹⁹F NMR (376 MHz, CDCl₃): *δ* -57.85 (3F). HMRS (DCl-CH₄⁺): Calc. pour C₃₁H₃₃AuF₃N₂O₁₀S: 879,1437 trouvé 879,1474.

**Complexe 37.** À partir de tétraacétate de thio-β-D-glucose (62 mg, 0,17 mmol) et complexe **22** (91 mg, 0,15 mmol). Solide blanc (93 mg, 59%). ¹H NMR (400 MHz, CDCl₃): *δ* 8.22 (d, *J* = 1.5 Hz, 2H), 8.02 (s, 1H), 7.56-7.37 (m, 5H), 7.30 (d, *J* = 2.0 Hz, 1H), 7.18 (d, *J* = 1.9 Hz, 1H), 5.75-5.51 (m, 2H), 5.11 (ddd, *J* = 9.3, 7.4, 1.7 Hz, 1H), 5.02-4.93 (m, 3H), 4.15 (dd, *J* = 12.2, 5.0 Hz, 1H), 4.03 (dd, *J* = 12.2, 2.4 Hz, 1H), 3.68 (ddd, *J* = 10.0, 5.0, 2.4 Hz, 1H), 2.03 (s, 3H), 2.01 (s, 3H), 1.97 (s, 3H), 1.96 (s, 3H). ¹³C NMR (101 MHz, CDCl₃): *δ* 183.69 (1C), 170.76 (1C), 170.25 (1C), 169.78 (1C), 169.55 (1C), 140.33 (1C), 134.62 (1C), 133.25 (2C), 129.33 (2C), 129.10 (1C), 128.18 (2C), 125.21 (1C), 125.18 (1C), 122.83 (1C), 121.92 (1C), 121.28 (1C), 120.64 (2C), 83.03 (1C), 77.46 (1C), 75.71 (1C), 74.26 (1C), 69.01 (1C), 62.91 (1C), 55.50 (1C), 21.03 (1C), 20.70 (1C), 20.65 (2C). ¹⁹F NMR (376 MHz, CDCl₃): *δ* -62.78 (6F). HMRS (DCl-CH₄⁺): Calc. pour C₃₂H₃₂AuF₆N₂O₉S: 931.1367 trouvé 931,1398.

**Complexe 38.** À partir de tétraacétate de thio-β-D-glucose (36 mg, 0,1 mmol) et complexe **23** (50 mg, 0,09 mmol). Solide blanc (72 mg, 91%). ¹H NMR (400 MHz, CDCl₃): *δ* 7.94 (ddd, J = 8.1, 2.1, 0.9 Hz, 1H), 7.67 (dd, J = 8.2 Hz, 1H), 7.49-7.35 (m, 7H), 7.22 (d, J = 2.0 Hz, 1H), 7.08 (d, J = 2.0 Hz, 1H), 5.53-5.49 (m, 2H), 5.10 (ddd, J = 9.5, 8.5, 2.1 Hz, 1H), 5.02-4.84 (m, 3H), 4.13 (dd, J = 12.2, 4.9 Hz, 1H), 4.03 (dd, J = 12.2, 2.4 Hz, 1H), 3.66 (ddd, J = 10.1, 4.9, 2.4 Hz, 1H), 2.04 (s, 3H), 2.02 (s, 3H), 1.99 (s, 3H), 1.97 (s, 3H). ¹³C NMR (101 MHz, CDCl₃): *δ* 182.91 (1C), 170.75 (1C), 170.22 (1C), 169.84 (1C), 169.59 (1C), 149.52 (1C), 140.26 (1C), 134.88 (1C), 131.15 (1C), 129.19 (2C), 128.89 (1C), 128.18 (2C), 123.57 (1C), 121.48 (1C), 121.45 (1C), 121.14 (1C), 120.33 (1C), 117.36 (1C), 83.03 (1C), 77.51 (1C), 75.60 (1C), 74.28 (1C), 68.93 (1C), 62.88 (1C), 55.34 (1C), 21.11 (1C), 20.74 (1C), 20.67 (1C), 20.65 (1C). ¹⁹F NMR (376 MHz, CDCl₃): *δ* -57.78 (3F). HMRS (DCl-CH₄⁺): Calc. pour C₃₁H₃₃AuF₃N₂O₁₀S: 879,1456 trouvé 879,1474.

**Complexe** 39. À partir de tétraacétate de thio-β-D-glucose (29 mg, 0,08 mmol) et complexe **27** (39 mg, 0,07 mmol). Solide blanc (40 mg, 65%). ¹H NMR (400 MHz, CDCl₃): *δ* 8.20 (d, J = 1.5 Hz, 2H), 7.99 (s, 1H), 7.31 (d, J = 2.0 Hz, 1H), 7.27 (d, J = 2.1 Hz, 1H), 5.34 (hept, J = 6.8 Hz, 1H), 5.13-5.02 (m, 2H), 4.99-4.92 (m, 2H), 4.21 (dd, J = 12.2, 5.0 Hz, 1H), 4.06 (dd, J = 12.2, 2.5 Hz, 1H), 3.69 (ddd, J = 9.5, 5.0, 2.4 Hz, 1H), 2.03 (s, 3H), 2.01 (s, 3H), 2.00 (s, 3H), 1.97 (s, 3H), 1.63 (d, J = 6.8 Hz, 6H). ¹³C NMR (101 MHz, CDCl₃): *δ* 182.46 (1C), 170.76 (1C), 170.23 (1C), 169.62 (2C), 140.58 (1C), 133.14 (2C), 125.17 (2C), 123.29 (1C), 122.67 (1C), 121.94 (1C), 121.02 (1C), 118.24 (1C), 82.99 (1C), 77.52 (1C), 75.57 (1C), 74.23 (1C), 68.98 (1C), 62.97 (1C), 54.19 (1C), 23.34 (1C), 23.26 (1C), 20.99 (1C), 20.78 (1C), 20.64 (2C). ¹⁹F NMR (376 MHz, CDCl₃): *δ* -62.78 (6F). HMRS (DCl-CH₄⁺): Calc. pour C₂₈H₃₂AuF₆N₂O₉S: 883,1369 trouvé 883,1398.

**Complexe 40.** À partir de tétraacétate de thio-β-D-glucose (47 mg, 0,13 mmol et complexe **29** (52 mg, 0,12 mmol). Solide blanc (75 mg, 82%). ¹H NMR (400 MHz, CDCl₃): *δ* 7.79-7.66 (m, 2H), 7.32-7.25 (m, 2H), 7.18 (d, J = 2.0 Hz, 1H), 7.15 (d, J = 2.1 Hz, 1H), 5.23 (hept, J = 6.8 Hz, 1H), 5.11 (, J = 9.2, 7.4, 1.7 Hz, 1H), 5.01-4.84 (m, 3H), 4.20 (dd, J = 12.2, 4.8 Hz, 1H), 4.05 (dd, J = 12.2, 2.4 Hz, 1H), 3.68 (ddd, J = 10.0, 4.8, 2.4 Hz, 1H), 2.06 (s, 3H), 2.03 (s, 6H), 2.02 (s, 3H), 1.57 (d, J = 6.8 Hz, 6H). ¹³C NMR (101 MHz, CDCl₃): *δ* 181.62 (1C), 170.74 (1C), 170.30 (1C), 169.80 (1C), 169.57 (1C), 163.72 (1C), 161.24 (1C), 126.64 (1C), 126.55 (1C), 121.50 (1C), 116.92 (1C), 116.77 (1C), 116.54 (1C), 83.01 (1C), 77.71 (1C), 75.46 (1C), 74.19 (1C), 68.91 (1C), 62.92 (1C), 53.62 (1C), 23.39 (1C), 23.30 (1C,), 21.15 (1C), 20.81 (1C), 20.67 (1C), 20.64 (1C). ¹⁹F NMR (376 MHz, CDCl₃): *δ* -111.62 (1F). HMRS (DCl-CH₄⁺): Calc. pour C₂₆H₃₃AuFN₂O₉S: 765,1528 trouvé 765,1556.

### H. Protocole de synthèse des précurseurs des complexes d'or(I) 41 et 42 et de ces complexes

### 1-{4-[(trifluorométhyl)sulfanyl]phényl}-1H-imidazole (1') de formule (A) :

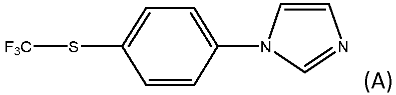

Dans un ballon sous pression, de l'imidazole (264,15 mg, 3,88 mmol), du 1-bromo-4-(trifluorométhylsulfanyl)benzène (500 mg, 1,94 mmol), du K₂CO₃ (268,11 mg, 1,94 mmol) et une quantité catalytique de CuSO₄ ont été mélangés à 180°C pendant 48 h. Après refroidissement à température ambiante, le produit brut a été extrait avec du MeOH et filtré puis le solvant a été évaporé sous pression réduite. Le produit a été purifié par chromatographie sur colonne sur un gel de silice avec un mélange de CH₂Cl₂ et de MeOH (90/10) comme éluant pour donner un solide jaune (382,8 mg, 80% de rendement). ¹H NMR (400 MHz, CD₃CN) *δ* 8.08-7.99 (m, 1H, H2), 7.86-7.75 (m, 2H, H8), 7.67-7.57 (m, 2H, H7), 7.55-7.45 (m, 1H, H5), 7.21-7.12 (m, 1H, H4).¹³C NMR (101 MHz, CD₃CN) *δ* 139.64 (1C, C2), 137.97 (2C, C8), 135.65 (1C, C9), 130.52 (1C, C6), 129.75 (1C, C*F*₃, q, J = 307.1 Hz), 121.83 (1C, C4), 121.63 (2C, C7), 117.30 (1C, C5).¹⁹F NMR (376 MHz, CDCl₃) *δ* -42.70 (3F, C*F₃*). HMRS (ES⁺): Calc. pour C₁₀H₈F₃N₂S 245,0360 trouvé 245,0361 [M+H⁺].

### Chlorure de 3-Benzyl-1-{4-[(trifluorométhyl)sulfanyl]phényl}-1H-imidazol-3-ium (6') de formule (B)

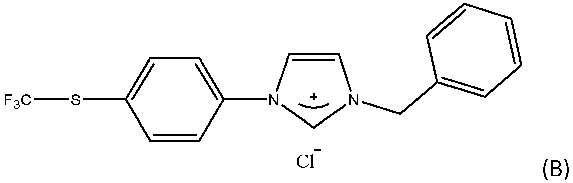

Sous atmosphère d'azote, **1'** (130 mg, 0,53 mmol) et du chlorure de benzyle (0,07 mL, 0,61 mmol) ont été dissous dans du CH₃CN sec (10 mL) puis le mélange a été agité à 80°C pendant 5 jours. La solution a ensuite été évaporée, le solide résultant a été lavé avec de l'éther et séché sous vide pour donner un solide blanc (130,3 mg, 66% de rendement). ¹H NMR (400 MHz, CDCl₃) *δ* 11.61 (t, J = 1.6 Hz, 1H, H2), 8.24 (t, J = 1.9 Hz, 1H, H5), 8.04-7.92 (m, 2H, H7), 7.73 (t, J = 1.8 Hz, 1H, H4), 7.72-7.64 (m, 2H, H8), 7.61-7.46 (m, 2H, H12), 7.27-7.15 (m, 3H, H13, H14), 5.68 (s, 2H, H10). ¹³C NMR (101 MHz, CDCl₃) *δ* 137.92 (2C, C8), 136.28 (1C, C2), 136.12 (1C, C9), 132.97 (1C, C6), 129.49 (1C, C11), 129.30 (2C, C7), 129.11 (2C, C13), 129.00 (1C, *C*F₃, q, J = 308.7 Hz), 126.57 (1C, C4), 123.27 (1C, C5), 122.35 (2C, C12), 121.08 (1C, C14), 53.42 (1C, C10). ¹⁹F NMR (376 MHz, CDCl₃) *δ* -42.17 (3F, C*F₃*). HMRS (ES⁺): Calc. pour C₁₇H₁₄N₂F₃S 335,0830 trouvé 335,0838 [M-Cl⁻].

### Bromure de 3-Isopropyl-1-(4-((trifluorométhyl)thio)phényl)-1H-imidazol-3-ium bromide (11') de formule (C)

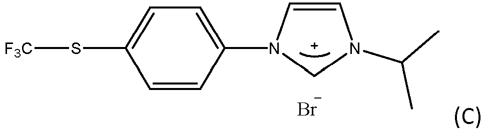

Dans un ballon sous pression, **1'** (125 mg, 0,59 mmol) a été dissous dans du 2-bromopropane (3 mL) et le mélange a été agité à 80°C une semaine. Le solvant a été évaporé sous pression réduite, et le solide lavé avec de l'éther diéthylique et séché pour donner un solide jaune (58,2 mg, 27% de rendement). ¹H NMR (300 MHz, CD₃CN) *δ* 10.32 (dt, J = 1.8, 0.9 Hz, 1H, H2), 8.13-8.01 (m, 3H, H4, H7), 7.98-7.91 (m, 2H, H8), 7.88 (t, J = 1.9 Hz, 1H, H5), 4.89 (qq, J = 6.7 Hz, 1H, H10), 1.65 (d, J = 6.7 Hz, 6H, H11). ¹³C NMR (75 MHz, CD₃CN) *δ* 137.83 (2C, C8), 137.24 (1C, C2), 134.92 (1C, C6), 129.62 (q, J = 307.2 Hz, 1C, *C*F₃), 125.55 (1C, C9), 123.41 (2C, C7), 121.63 (1C, C4), 121.19 (1C, C5), 53.94 (1C, C10), 21.99 (2C, C11). ¹⁹F NMR (282 MHz, CD₃CN) δ -43.38 (3F, C*F₃*). HMRS (ES⁺): Calc. pour C₁₃H₁₄F₃N₂S 287,0830 trouvé 287,0830 [M-Br⁻].

### Complexe 41 de formule (D)

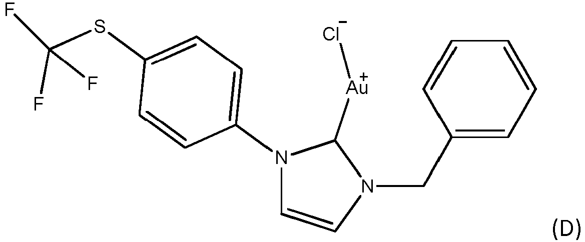

Sous atmosphère d'azote, 6' (80 mg, 0,22 mmol) et Ag₂O (27,04 mg, 0,12 mmol) ont été dessous dans du CH₂Cl₂ sec, le mélange a été agité à température ambiante pendant 18 h. Au(SMe₂)Cl (69,8 mg, 0,24 mmol) a été ajouté et mélangé pendant 2 h. La solution a été filtrée à travers un tampon de célite et le solvant a été éliminé sous pression réduite pour donner un solide blanc (119,8 mg, 98% de rendement). ¹H NMR (400 MHz, CD₃CN) *δ* 7.88 (s, 4H, H7, H8), 7.54-7.27 (m, 7H, H4, H5, H12, H13, H14), 5.48 (s, 2H, H10). ¹³C NMR (101 MHz, CD₃CN) *δ* 170.32 (1C, C2), 141.61 (1C, C6), 137.27 (2C, C13), 135.99 (C11), 129.66 (q, J = 307.1 Hz, 1C, *C*F₃), 128.93 (2C, C8), 128.49 (1C, C9), 127.94 (2C, C12), 126.37 (2C, C7), 124.70 (1C C14), 122.51 (1C, C5), 122.31 (1C, C4), 54.95 (1C, C10). ¹⁹F NMR (376 MHz, CD₃CN) δ -43.37 (3F, C*F₃*). HMRS (ES⁺): Calc. pour C₁₉H₁₆AuF₃N₃S 572,0683 trouvé 572,0682 [M-Cl⁻+CH₃CN].

### Complexe 42 de formule (E)

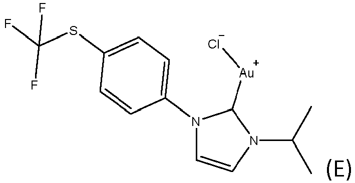

Sous atmosphère d'azote, 9' (47,7 mg, 0,13 mmol) et Ag₂O (14,79 mg, 0,064 mmol) ont été dessous dans du CH₂Cl₂ sec, le mélange a été agité à température ambiante pendant 18 h. Au(SMe₂)Cl (41,53 mg, 0,14 mmol) a été ajouté et mélangé pendant 2 h. La solution a été filtrée à travers un tampon de célite et le solvant a été éliminé sous pression réduite pour donner un solide blanc (61,9 mg, 92% de rendement). ¹H NMR (400 MHz, CDCl₃) *δ* 7.94-7.66 (m, 4H, H7, H8), 7.27 (d, J = 2.1 Hz, 1H, H5), 7.23 (d, J = 2.1 Hz, 1H, H4), 5.26 (qq, J = 6.8 Hz, 1H, H10), 1.57 (d, J = 6.8 Hz, 6H, H11). ¹³C NMR (101 MHz, CDCl₃) *δ* 169.54 (1C, C2), 141.18 (1C, C9), 137.36 (2C, C8), 129.22 (q, J = 308.4 Hz, 1C, *C*F₃), 125.67 (2C, C7), 125.59 (1C, C6), 121.67 (1C, C5), 117.77 (1C, C4), 54.46 (1C, C10), 23.29 (2C, C11). ¹⁹F NMR (376 MHz, CDCl₃) δ -42.11 (3F, C*F₃*). HMRS (ES⁺): Calc. pour C₁₅H₁₆AuF₃N₃S 524,0683 trouvé 524,0679 [M-Cl⁻+CH₃CN].

### II. Activité anti-leishmanienne et cytotoxicité des complexes de l'invention.

### II.1. Activité anti-leishmanienne sur des amastigotes axéniques de L. infantum.

Des promastigotes de *L. infantum* (MHOM/MA/67/ITMAP-263, CNR Leishmania, Montpellier, France, exprimant une activité luciférase) ont été cultivés dans un milieu RPMI 1640 supplémenté avec 10% de sérum de veau fœtal (FCS), 2 mM de L-glutamine et des antibiotiques (100 U/mL de pénicilline et 100 µg/mL de streptomycine) et récoltées en phase logarithmique de croissance par centrifugation à 900 g pendant 10 min. Le surnageant a été retiré avec précaution et a été remplacé par le même volume de milieu complet RPMI 1640 à pH 5,4 et incubé pendant 24 h à 24°C. Les promastigotes ont ensuite été incubés (incubateur humide à 5% de CO₂) pendant 24 h à 37°C conduisant à leur transformation en amastigotes axéniques.

Les amastigotes de *L. infantum* ont été incubés à une densité de 2 x 10⁶ parasites/ml dans des plaques stériles à 96 puits avec diverses concentrations de complexes dissous dans du DMSO (concentration finale de DMSO inférieure à 0,5% v/v), en duplicat. Des contrôles appropriés [DMSO, amphotéricine B, miltéfosine, pentamidine et fexinidazole (Sigma Aldrich)] ont été ajoutés à chaque série d'expériences.

L'activité luciférase des amastigotes axéniques a ensuite été estimée, 80 µL de chaque puits ont été transférés sur des plaques blanches à 96 puits, du Steady Glow^{®} (Promega) a été ajouté selon les instructions du fabricant, et les plaques ont été incubées (37°C) pendant 2 min. La luminescence a été mesurée en utilisant un Microbeta Luminescence Counter (PerkinElmer).

La concentration inhibitrice 50% (CI50) a été définie comme la concentration de médicament nécessaire pour inhiber de 50% l'activité métabolique des amastigotes de *L. infantum* par rapport au contrôle. Les valeurs CI50 ont été calculées par analyse de régression non linéaire traitée sur des courbes dose-réponse, à l'aide du logiciel TableCurve 2D V5. Les valeurs CI50 représentent la moyenne de trois expériences indépendantes.

### II.2. Evaluation de la cytotoxicité.

L'évaluation de la cytotoxicité des complexes selon l'invention par test MTT sur la lignée cellulaire Thp1 (lignée cellulaire de monocytes humains) a été réalisée selon Mosmann avec de légères modifications. En bref, les cellules (0,77.10⁵ cellules/ml) dans 200 µL du milieu complet [RPMI 1640 supplémenté avec 10% de sérum de veau fœtal (FCS), 2 mM de L-glutamine et des antibiotiques (100 U/ml) pénicilline et 100 µg/ml de streptomycine)]+ PMA (50 ng/ml) ont été ensemencées dans chaque puits de plaques à 96 puits et incubées à 37°C sous atmosphère humidifiée à 5% de CO₂ avec 95% d'air. Après une incubation de 96 h, les plaques ont été lavées 3 fois avec du milieu et 100 µl de milieu ont été ajoutés. Diverses concentrations et contrôles appropriés ont ensuite été ajoutés (100 µl) et les plaques ont été incubées pendant 72 h à 37°C.

Chaque puits a ensuite été examiné au microscope pour détecter la formation possible de précipité avant l'aspiration du milieu. Une solution de MTT (0,5 mg/ml dans du RPMI, 100 µl) a ensuite été ajoutée à chaque puits. Les cellules ont été incubées pendant 2 h à 37°C. La solution de MTT a ensuite été éliminée et du DMSO (100 µl) a été ajouté pour dissoudre les cristaux de formazan résultants. Les plaques ont été secouées vigoureusement (300 tr/min) pendant 5 min.

L'absorbance a été mesurée à 570 nm avec un spectrophotomètre pour microplaques. Le DMSO a été utilisé comme blanc et la doxorubicine (Sigma Aldrich) comme contrôle positif. Les CC50 ont été calculés par analyse de régression non linéaire traitée sur des courbes dose-réponse, à l'aide du logiciel TableCurve 2D V5.

### II.3. Résultats.

Le pouvoir anti-leishmanien des proligands **6-20** et des complexes d'or(I) **21-40** a été évalué *in vitro* sur le stade amastigote axénique de *Leishmania infantum,* (correspondant au stade responsable de la maladie chez l'Homme) en déterminant leurs concentrations inhibitrices à 50% (CI50) et comparé à trois médicaments de référence anti-leishmania différents (amphotéricine B, pentamidine et miltéfosine) et au candidat-médicament fexinidazole.

Afin d'évaluer leur sélectivité, la cytotoxicité de ces complexes a été évaluée *in vitro* sur la lignée cellulaire de leucémie monocytaire aiguë humaine Thp1 et les valeurs CC50 correspondantes ont été comparées à celles du médicament de référence doxorubicine donnant accès à des indices de sélectivité (SI = CC50/CI50). Les résultats sont résumés dans le Tableau 2.

Les précurseurs de carbène étaient inactifs avec des valeurs de CI50 supérieures à 50 µM, à l'exception du sel d'imidazolium 17 qui était modérément actif mais plutôt toxique.

Au contraire, tous les complexes d'or(I) **21-40** étaient actifs contre la forme amastigote axénique cliniquement pertinente de *L. infantum,* avec des valeurs CI50 dans la gamme nanomolaire avec des valeurs CI50 de 0,77 à 0,09 µM, sauf **22** qui affiche une valeur CI50 de 1,53 µM.

En ce qui concerne l'activité anti-leishmanienne pour les complexes d'or(I) portant un groupe benzyle, il semble que cette série soit active avec des valeurs CI50 dans la gamme nanomolaire également (CI50 = 0,25-0,77 µM) sauf le complexe **22** qui affiche une valeur CI50 de 1,53 µM.

Les valeurs de CI50 pour les complexes portant un groupe isopropyle étaient 2 à 3 fois meilleures que les valeurs de CI50 pour les mêmes complexes mais portant un groupe benzyle avec des valeurs de CI50 dans la gamme nanomolaire (CI50 = 0,13-0,5 µM). Ainsi, l'introduction d'un groupe isopropyle au lieu d'un groupe benzyle semble favoriser l'activité anti-leishmanienne.

Fait intéressant, les composés avec introduction d'un groupe quinolyle affichaient de meilleures valeurs de CI50 que les analogues isopropyle et benzyle avec une activité anti-leishmanienne de 0,09 µM à 0,12 µM, sauf pour le complexe **32** qui affiche une valeur CI50 de 0,19 µM mais qui est toujours meilleure que le complexe correspondant portant un groupe isopropyle (complexe **27** avec une CI50 de 0,41 µM). Ces dérivés ont montré une activité équivalente à celle de l'amphotéricine B de référence avec une CI50 de 0,075 µM.

Tous les complexes d'or(I) (sauf 22) ont montré une activité identique ou meilleure par rapport aux médicaments anti-leishmaniens miltéfosine (CI50 = 0,71 µM), pentamidine (CI50 = 10,54 µM) et le candidat-médicament fexinidazole (CI50 = 3,4 µM).

Afin d'évaluer la cytotoxicité des complexes d'or(I), ils ont été testés *in vitro* sur la lignée cellulaire Thp1.

Les résultats biologiques ont montré que les complexes d'or(I) présentaient une faible cytotoxicité sur la lignée cellulaire humaine Thp1 avec des valeurs CC50 allant de 12,21 à 88,79 µM par rapport aux valeurs des médicaments de référence amphotéricine B (CC50 = 10,49 µM) et miltéfosine (CC50 = 35,02 µM). La pentamidine et le fexinidazole étaient moins cytotoxiques avec des valeurs CC50 supérieures à 50 µM et à 355 µM respectivement.

Les complexes d'or(I) portant un groupe benzyle ou isopropyle étaient les moins cytotoxiques (CC50 = 14,93-88,79 µM) mais les complexes avec un groupe quinolyle étaient encore moins cytotoxiques (CC50 = 12,21-53,02 µM).

Tous les composés synthétisés ont montré de très bons indices de sélectivité (SI = 47,09-474,17) avec la meilleure valeur pour **34.** Ces valeurs SI étaient meilleures que pour la pentamidine (SI>4,74) et dans la même gamme pour les autres médicaments de référence (SI = 139,87 pour amphotéricine B, SI = 53,06 pour la miltéfosine et > 104,41 pour le candidat-médicament fexinidazole).

Les complexes **36-40** ont montré une très bonne activité anti-leishmanienne avec une CI50 allant de 0,14 à 0,30 µM. Ces complexes avaient une faible cytotoxicité avec CC50 allant de 3,99 à 12,46 µM, dans les mêmes valeurs que l'amphotéricine B. Tous ces résultats préliminaires pour les complexes **36-40** conduisent à de bons indices de sélectivité allant de 13 à 54, équivalents à celui de la miltéfosine.

**Tableau 2**

| **Composés ou Complexes** | ***Amastigotes axéniques de L. infantum* CI₅₀ (µM)^{a}** | **Thp1 CC₅₀ (µµM)^{a}** | **Indice de sélectivité CC₅₀/IC₅₀ SI** |
|---|---|---|---|
| **6** | >50 | - | - |
| **7** | >50 | - | - |
| **8** | >50 | - | - |
| **9** | >50 | - | - |
| **10** | >50 | - | - |
| **11** | >50 | - | - |
| **12** | >50 | - | - |
| **13** | >50 | - | - |
| **14** | >50 | - | - |
| **15** | >50 | - | - |
| **16** | >50 | - | - |
| **17** | 14,50 | 44,48 | 3,07 |
| **18** | >50 | - | - |
| **19** | >50 | - | |
| **20** | >50 | - | - |
| **21** | 0,28 | 18,89 | 67,85 |
| **22** | 1,53 | 71,88 | 47,09 |
| **23** | 0,77 | 48,08 | 62,55 |
| **24** | 0,25 | 18,29 | 71,89 |
| **25** | 0,33 | 20,88 | 63,21 |
| **26** | 0,13 | 14,93 | 118,53 |
| **27** | 0,41 | 49,65 | 119,72 |
| **28** | 0,50 | 88,79 | 178.52 |
| **29** | 0,25 | 45,51 | 178,50 |
| **30** | 0,14 | 18,97 | 133,82 |
| **31** | 0,10 | 13,38 | 136,42 |
| **32** | 0,19 | 12,20 | 65,03 |
| **33** | 0,09 | 12,21 | 129,70 |
| **34** | 0,11 | 53,02 | 474,17 |
| **35** | 0,12 | 15,47 | 130,05 |
| **36** | 0,23 | 12,46 | 53,92 |
| **37** | 0,14 | 4,40 | 30,63 |
| **38** | 0,30 | 6,94 | 23,15 |
| **39** | 0,27 | 5,13 | 18,79 |
| **40** | 0,30 | 3,99 | 13.43 |
| Amphotéricine B^{b} | 0,075 | 10,49 | 139,87 |
| Miltéfosine^{b} | 0,71 | 35,02 | 49,32 |
| Pentamidine^{b} | 10,54 | >50 | >4,74 |
| Fexinidazole^{b} | 3,4 | >355 | >104,41 |
| Doxorubicine^{c} | - | 0,70 | - |

| | | | |
|---|---|---|---|
| ^{a} Moyenne de trois expériences indépendantes. ^{b} Amphotéricine B, miltéfosine et fexinidazole ont été utilisés comme médicaments de référence ou médicaments candidats anti-leishmaniens. ^{c} Doxorubicine a été utilisée comme contrôle positif de cytotoxicité. | | | |

Les complexes d'or(I) **41** et **42** ont été testés de la même manière mais lors d'expériences distinctes. Les résultats pour ces complexes sont résumés dans le Tableau 3. Ces complexes montrent des activités et sélectivités très proches de la miltéfosine, meilleures que la pentamidine et un peu moindres que l'amphotéricine B.

**Tableau 3**

| **Complexes** | ***Amastigotes axéniques de L. infantum* CI₅₀ (µM)^{a}** | **Thp1 CC₅₀ (µM)^{a}** | **Indice de sélectivité CC₅₀/IC₅₀ SI** |
|---|---|---|---|
| **41** | 0,34 ± 0,03 | 15,84 ± 2,86 | 46,58 |
| **42** | 0,15 ± 0,04 | 16,24 ± 3,27 | 108,27 |
| Amphotéricine B^{b} | 0,06 ± 0,01 | 13,13 ± 0,92 | 218,83 |
| Miltéfosine^{b} | 0,46 ± 0,04 | 35,02 ± 2,98 | 76,13 |
| Pentamidine^{b} | 1,36 ± 0,31 | 65.87±7.49 | 48.43 |
| Doxorubicine^{c} | - | 0,7 ± 0,06 | - |

| | | | |
|---|---|---|---|
| ^{a} Moyenne de trois expériences indépendantes. ^{b} Amphotéricine B et miltéfosine ont été utilisés comme médicaments de référence ou médicaments candidats anti-leishmaniens. ^{c} Doxorubicine a été utilisée comme contrôle positif de cytotoxicité. | | | |

### RÉFÉRENCES

[1] Paloque et al, 2015, « Synthesis, characterization, and antileishmanial activities of gold(I) complexes involving quinoline functionalized N-heterocyclic carbenes », European Journal of Medicinal Chemistry, vol. 94, pages 22-29.
[2] Zhang et al, 2018, « Synthesis, characterization, and antileishmanial activities of neutral N-heterocyclic carbenes gold(I) complexes », European Journal of Medicinal Chemistry, vol. 143, pages 1635-1643.
[3] Ouji et al, 2020, « Design, synthesis and efficacy of hybrid triclosan-gold based molecules on artemisinin-resistant Plasmodium falciparum and Leishmania infantum parasites », ChemistrySelect, vol. 5, pages 619-625.
[4] Boselli et al, 2015, « Synthesis, structures, and biological studies of heterobimetallic Au(I)-Ru(II) complexes involving N-heterocyclic carbene- based multidentate ligands », Organometallics, vol. 34, pages 1046-1055.
[5] Mora et al, 2019, « Recent advances in gold-NHC complexes with biological properties », Chem. Soc. Rev., vol. 48, pages 447-462.
[6] Delgado-Rebollo et al, 2019, « Coinage metal complexes bearing fluorinated N-heterocyclic carbene ligands », Journal of Organometallic Chemistry, vol. 898, 120856
[7] Price et al, 2017, « Some insights into the gold-catalysed A3-coupling reaction », Journal of Organometallic Chemistry, vol. 846, pages 251-262.
[8] Zhang et al, 2018 «Cationic and Neutral N -Heterocyclic Carbene Gold(I) Complexes: Cytotoxicity, NCI-60 Screening, Cellular Uptake, Inhibition of Mammalian Thioredoxin Reductase, and Reactive Oxygen Species Formation», ChemMedChem, vol. 13, pages 1218-1229.

## Revendications

1. Complexe d'or(I) répondant à l'une des formules (I) ou (II) suivantes : 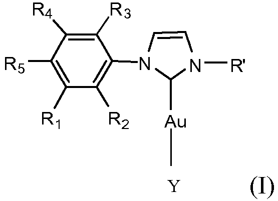 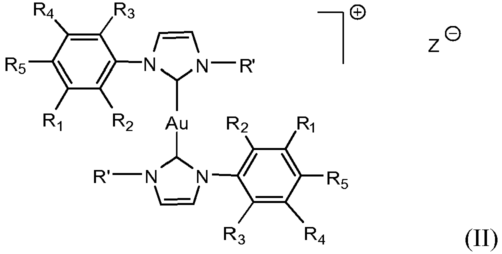 dans lesquelles
• R₁, R₂, R₃, R₄ et R₅, identiques ou différents, sont indépendamment choisis dans le groupe constitué par H, F, CₙF₂ₙ₊₁, OCₙF₂ₙ₊₁ et SCₙF₂ₙ₊₁ avec n égal à 1, 2, 3 ou 4, à condition qu'au moins un parmi R₁, R₂, R₃, R₄ et R₅ soit H et qu'au moins un autre parmi R₁, R₂, R₃, R₄ et R₅ soit choisi dans le groupe constitué par F, CₙF₂ₙ₊₁, OCₙF₂ₙ₊₁ et SCₙF₂ₙ₊₁ ;
• R' représente un groupement alkyle éventuellement substitué ou un groupement aryle éventuellement substitué ;
• Y représente un halogène ou un groupement -S-X avec X représentant un reste d'un ose à 5 ou 6 atomes ou un de ses dérivés ; et
• Z⁻ représente un anion halogènure, un anion nitrate (-NO₃⁻) ou un anion non coordinant,
à condition que, lorsque le complexe d'or(I) répond à la formule (I) et que R₁ = R₂ = R₄ = H, R₃ = R₅ = F et Y = Cl, R' n'est pas un 2,4-difluorophényle.

2. Complexe d'or(I) selon la revendication 1, caractérisé en que R₁, R₂, R₃, R₄ et R₅, identiques ou différents, sont indépendamment choisis dans le groupe constitué par H, F, CF₃, OCF₃ et SCF₃, à condition qu'au moins un parmi R₁, R₂, R₃, R₄ et R₅ soit H et qu'au moins un autre parmi R₁, R₂, R₃, R₄ et R₅ soit choisi dans le groupe constitué par F, CF₃, OCF₃ et SCF₃.

3. Complexe d'or(I) selon la revendication 1 ou 2, **caractérisé en ce que** ledit R' est un isopropyle.

4. Complexe d'or(I) selon la revendication 1 ou 2, **caractérisé en ce que** ledit R' est un mésityle, un benzyle, un quinolyle, un méthyl-pyrrole, un sulfure de méthyle phényle ou un bipyridine.

5. Complexe d'or(I) selon l'une quelconque des revendications 1 à 4, **caractérisé en ce que** ledit Y est un chlore.

6. Complexe d'or(I) selon l'une quelconque des revendications 1 à 4, **caractérisé en ce que** ledit groupement -S-X est le 2,3,4,6-Tetra-O-acétyl-1-thio-β-D-glucopyranosato.

7. Composition pharmaceutique comprenant, en tant que principe actif, un complexe d'or(I) selon l'une quelconque des revendications 1 à 6 et/ou le chloro-[1,3-bis(2,4-difluorophényle)imidazoline-2-ylidène] or(I) et un véhicule pharmaceutiquement acceptable.

8. Complexe d'or(I) selon l'une quelconque des revendications 1 à 6 et/ou chloro-[1,3-bis(2,4-difluorophényle)imidazoline-2-ylidène] or(I), pour une utilisation thérapeutique comme agent anti-oxydant, comme agent anti-cancéreux, comme agent anti-bactérien et/ou comme agent anti-parasitaire.

9. Complexe d'or(I) pour utilisation selon la revendication 8, ledit complexe d'or(I) étant utile comme agent anti-leishmanien.

10. Complexe d'or(I) selon l'une quelconque des revendications 1 à 6 et/ou chloro-[1,3-bis(2,4-difluorophényle)imidazoline-2-ylidène] or(I) ou une composition pharmaceutique selon la revendication 7, pour utilisation dans le traitement et/ou la prévention d'une leishmaniose.

## Patentansprüche

1. Gold(I)-Komplex, der einer der folgenden Formeln (I) oder (II) entspricht: 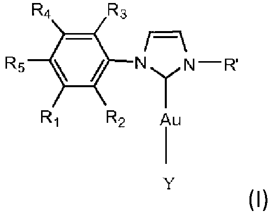 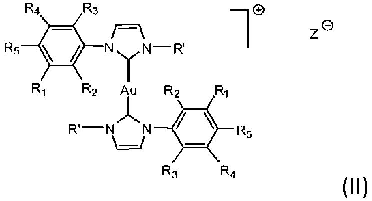 wobei:
• R₁, R₂, R₃, R₄ und R₅, identisch oder unterschiedlich, unabhängig voneinander aus der Gruppe ausgewählt sind, die von H, F, CₙF₂ₙ₊₁, OCₙF₂ₙ₊₁ und SCₙF₂ₙ₊₁ gebildet ist, wobei n gleich 1, 2, 3 oder 4 ist, sofern mindestens eines von R₁, R₂, R₃, R₄ und R₅ entweder H ist und mindestens ein anderes von R₁, R₂, R₃, R₄ und R₅ aus der Gruppe ausgewählt ist, die von F, CₙF₂ₙ₊₁, OCₙF₂ₙ₊₁ und SCₙF₂ₙ₊₁ gebildet ist;
• R' eine eventuell substituierte Alkylgruppe oder eine eventuell substituierte Arylgruppe darstellt;
• Y ein Halogen oder eine -S-X-Gruppe darstellt, wobei X einen Zuckerrest mit 5 oder 6 Kohlenstoffatomen oder eines seiner Derivate darstellt; und
• Z⁻ ein Halogenidanion, ein Nitratanion (-NO₃⁻) oder ein nicht koordinierendes Anion darstellt,
sofern, wenn der Gold(I)-Komplex der Formel (I) entspricht und R₁ = R₂ = R₄ = H, R₃ = R₅ = F und Y = Cl ist, R' kein 2,4-Difluorphenyl ist.

2. Gold(l)-Komplex nach Anspruch 1, **dadurch gekennzeichnet, dass** R₁, R₂, R₃, R₄ und R₅, identisch oder unterschiedlich, unabhängig voneinander aus der Gruppe ausgewählt sind, die von H, F, CF₃, OCF₃ und SCF₃ gebildet ist, sofern mindestens eines von R₁, R₂, R₃, R₄ und R₅ entweder H ist und dass mindestens ein anderes von R₁, R₂, R₃, R₄ und R₅ aus der Gruppe ausgewählt ist, die von F, CF₃, OCF₃ und SCF₃ gebildet ist.

3. Gold(l)-Komplex nach Anspruch 1 oder 2, **dadurch gekennzeichnet, dass** R' ein Isopropyl ist.

4. Gold(l)-Komplex nach Anspruch 1 oder 2, **dadurch gekennzeichnet, dass** R' ein Mesityl, Benzyl, Quinolyl, Methylpyrrol, Methylphenylsulfid oder Bipyridin ist.

5. Gold(l)-Komplex nach einem der Ansprüche 1 bis 4, **dadurch gekennzeichnet, dass** Y ein Chlor ist.

6. Gold(l)-Komplex nach einem der Ansprüche 1 bis 4, **dadurch gekennzeichnet, dass** die -S-X-Gruppe 2,3,4,6-Tetra-O-acetyl-1-thio-β-D-glucopyranosat ist.

7. Pharmazeutische Zusammensetzung, die als Wirkstoff einen Gold(l)-Komplex nach einem der Ansprüche 1 bis 6 und/oder Chlor-[1,3-bis(2,4-difluorphenyl)imidazolin-2-yliden]-Gold(l) und einen pharmazeutisch akzeptablen Träger umfasst.

8. Gold(l)-Komplex nach einem der Ansprüche 1 bis 6 und/oder Chlor-[1,3-bis(2,4-difluorphenyl)imidazolin-2-yliden]-Gold(l) für eine therapeutische Verwendung als Antioxidationsmittel, als Mittel gegen Krebs, als antibakterielles Mittel und/oder als Mittel gegen Parasiten.

9. Gold(l)-Komplex zur Verwendung nach Anspruch 8, wobei der Gold(l)-Komplex als Wirkstoff gegen Leishmaniose nützlich ist.

10. Gold(l)-Komplex nach einem der Ansprüche 1 bis 6 und/oder Chlor-[1,3-bis(2,4-difluorphenyl)imidazolin-2-yliden]-Gold(l) oder eine pharmazeutische Zusammensetzung nach Anspruch 7 zur Verwendung bei der Behandlung von und/oder Vorbeugung gegen Leishmaniose.

## Claims

1. Gold (l) complex corresponding to one of the following formulae (I) or (II): 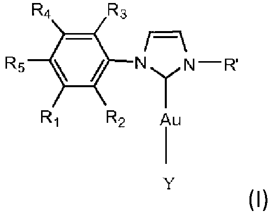 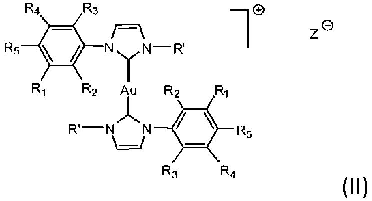 wherein
• R₁, R₂, R₃, R₄ and R₅, identical or different, are independently selected from the group consisting of H, F, CₙF₂ₙ₊₁, OCₙF₂ₙ₊₁ and SCₙF₂ₙ₊₁ with n equal to 1, 2, 3 or 4, provided that at least one amongst R₁, R₂, R₃, R₄ and R₅ is H and at least another one amongst R₁, R₂, R₃, R₄ and R₅ is chosen from the group consisting of F, CₙF₂ₙ₊₁, OCₙF₂ₙ₊₁ and SCₙF₂ₙ₊₁;
• R' represents an optionally substituted alkyl group or an optionally substituted aryl group;
• Y represents a halogen or an -S-X group with X representing a residue of a 5- or 6-atom ose or a derivative thereof; and
• Z ⁻ represents a halide anion, a nitrate anion (-NO₃⁻) or a non-coordinating anion,
provided that, when the gold (l) complex is of the formula (I) and R₁ = R₂ = R₄ = H, R₃ = R₅ = F and Y = Cl, R' is not a 2,4-difluorophenyl.

2. Gold (l) complex according to claim 1, **characterised in that** R₁, R₂, R₃, R₄ and R₅, identical or different, are independently chosen from the group consisting of H, F, CF₃, OCF₃ and SCF₃, provided that at least one of R₁, R₂, R₃, R₄ and R₅ is H and that at least another one of R₁, R₂, R₃, R₄ and R₅ is chosen from the group consisting of F, CF₃, OCF₃ and SCF₃.

3. Gold (l) complex according to claim 1 or 2, **characterised in that** said R' is an isopropyl.

4. Gold (l) complex according to claim 1 or 2, **characterised in that** said R' is a mesityl, a benzyl, a quinolyl, a methyl-pyrrole, a methyl phenyl sulphide or a bipyridine.

5. Gold (l) complex according to any one of claims 1 to 4, **characterised in that** said Y is a chlorine.

6. Gold (l) complex according to any one of claims 1 to 4, **characterised in that** said -S-X group is 2,3,4,6-tetra-O-acetyl-1-thio-β-D-glucopyranosato.

7. Pharmaceutical composition comprising, as the active ingredient, a gold (l) complex according to any one of claims 1 to 6 and/or chloro-[1,3-bis(2,4-difluorophenyl)imidazoline-2-ylidene] gold (l) and a pharmaceutically-acceptable carrier.

8. Gold (l) complex according to any one of claims 1 to 6 and/or chloro-[1,3-bis(2,4-difluorophenyl)imidazoline-2-ylidene] gold (l), for therapeutic use as an antioxidant agent, as an anti-cancer agent, as an antibacterial agent and/or as an anti-parasitic agent.

9. Gold (l) complex for use according to claim 8, said gold (l) complex being useful as an anti-leishmania agent.

10. Gold (l) complex according to any one of claims 1 to 6 and/or chloro-[1,3-bis(2,4-difluorophenyl)imidazoline-2-ylidene] gold (l) or a pharmaceutical composition according to claim 7, for use in the treatment and/or prevention of leishmaniasis.
